(19) Europäisches Patentamt
European Patent Office
Office européen des brevets

(11) Veröffentlichungsnummer: **0 266 308 B1**

(12) **EUROPÄISCHE PATENTSCHRIFT**

(45) Veröffentlichungstag der Patentschrift: **24.07.91**

(21) Anmeldenummer: **87810557.6**

(22) Anmeldetag: **25.09.87**

(51) Int. Cl.5: **C07D 487/14**, A61K 31/55, //(C07D487/14,243:00,241:00, 209:00)

(54) **Indolo-pyrazino-benzodiazepin-Derivate.**

(30) Priorität: **01.10.86 US 914028**

(43) Veröffentlichungstag der Anmeldung:
**04.05.88 Patentblatt 88/18**

(45) Bekanntmachung des Hinweises auf die Patenterteilung:
**24.07.91 Patentblatt 91/30**

(84) Benannte Vertragsstaaten:
**AT BE CH DE ES FR GB GR IT LI LU NL SE**

(56) Entgegenhaltungen:
**EP-A- 0 001 585**

(73) Patentinhaber: **CIBA-GEIGY AG**
**Klybeckstrasse 141**
**CH-4002 Basel(CH)**

(72) Erfinder: **Wasley, Jan W. F.**
**55 Floral Street**
**Chatham New Jersey 07928(US)**

EP 0 266 308 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 1,3,4,16b-Tetrahydro-2H,10H-indolo[2,1-c]pyrazino[1,2-a][1,4]benzodiazepin-Derivate, die als Serotonin-2-Rezeptor-Antagonisten und als therapeutische Wirkstoffe zur Behandlung von Krankheiten, welche darauf ansprechen, Verwendung finden, Verfahren zu deren Herstellung, pharmazeutische Präparate, die diese Verbindungen enthalten, sowie ihre Verwendung zur Behandlung von Symptomen, Zuständen und Krankheiten bei Säugern, welche auf die Wirkung eines solchen Serotonin-Rezeptor-Antagonisten durch Verabreichung solcher Verbindungen oder eines pharmazeutischen Präparates, enthaltend solche Verbindungen, ansprechen.

In der EP-A1-001,585 sind bereits 1,3,4,14b-Tetrahydro-2H,10H-pyrazino[1,2-a]pyrrolo[2,1-c][1,4]-benzodiazepine offenbart worden. Gemäss jener Patentanmeldung weisen sie pharmakologische Eigenschaften auf, in erster Linie antidepressive, aber auch analgetische, antihistaminische und antiserotonergische Wirkungen. Hinweise auf Wirkungen auf das cardiovaskuläre System werden nicht gegeben. Ebenfalls fehlen Hinweise darauf, dass statt der viergliedrigen Heterocyclen, die in jener Patentanmeldung offenbart sind, eventuell auch fünfgliedrige Heterocyclen vorteilhafte pharmakologische Eigenschaften haben könnten.

Die Erfindung betrifft 1,3,4,16b-Tetrahydro-2H,10H-indolo[2,1-c]pyrazino[1,2-a][1,4]benzodiazepin-Derivate der Formel I,

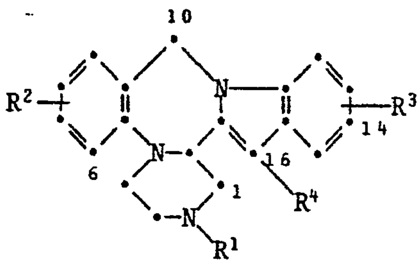

(I)

worin $R^1$ Wasserstoff, Niederalkyl, $C_3$-$C_7$-Alkenyl, welches über ein gesättigtes Kohlenstoffatom gebunden ist, $C_3$-$C_7$-Alkinyl, welches über ein gesättigtes Kohlenstoffatom gebunden ist, 3 bis 7-gliedriges Cycloalkyl, oder $C_2$-$C_7$-Alkyl, welches durch einen Substituenten ausgewählt aus der Gruppe bestehend aus Hydroxy, Amino, N-Mononiederalkylamino und N,N-Diniederalkylamino substituiert ist, wobei diese Substituenten vom Ringstickstoffatom durch mindestens 2 Kohlenstoffatome getrennt sind, bedeutet; oder worin $R^1$ Niederalkyl, welches durch einen Substituenten ausgewählt aus der Gruppe bestehend aus 3 bis 7-gliedrigem Cycloalkyl, Carboxy, Niederalkoxycarbonyl, Carbamoyl, N-Mononiederalkylcarbamoyl, N,N-Diniederalkylcarbamoyl und Niederalkanoyl substituiert ist, bedeutet; oder worin $R^1$ Niederalkyl, welches durch Phenyl oder Benzoyl substituiert ist, bedeutet, wobei jedes dieser Phenyl- oder Benzoylradikale unsubstituiert oder durch bis zu drei Substituenten ausgewählt aus der Gruppe bestehend aus Niederalkyl, Niederalkoxy, Niederalkylthio, Halogen und Trifluormethyl substituiert ist; worin $R^2$ und $R^3$, unabhängig voneinander, für Wasserstoff, Niederalkyl, Hydroxy, Niederalkoxy, Halogen oder Trifluormethyl stehen und $R^4$ für Wasserstoff, Niederalkyl, Hydroxyniederalkyl, Carboxy, Niederalkoxycarbonyl, Carbamoyl, N-Mono- oder N,N-Diniederalkylcarbamoyl oder Formyl steht; Salze, insbesondere pharmazeutisch annehmbare Salze davon; und die 2-N-Oxide dieser Verbindungen, worin $R^1$ die vorstehend angegebenen Bedeutungen aufweist aber nicht Wasserstoff bedeutet.

Bevorzugt sind die Verbindungen der Formel I, worin $R^1$ Wasserstoff, Niederalkyl, $C_3$-$C_7$-Alkenyl, welches über ein gesättigtes Kohlenstoffatom gebunden ist, oder $C_2$-$C_7$-Alkyl, welches durch Hydroxy substituiert ist, bedeutet; worin $R^2$ and $R^3$, unabhängig voneinander, für Wasserstoff, Niederalkyl, Niederalkoxy, Halogen oder Trifluormethyl stehen und $R^4$ für Wasserstoff, Niederalkyl, Hydroxymethyl, Carboxy, Niederalkoxycarbonyl, Carbamoyl, oder N-Mono- oder N,N-Diniederalkylcarbamoyl steht; pharmazeutisch annehmbare Salze davon; und die 2-N-Oxide dieser Verbindungen, worin $R^1$ die vorstehend angegebenen Bedeutungen aufweist aber nicht Wasserstoff bedeutet.

Weiterhin bevorzugt sind die Verbindungen der Formel I, worin $R^1$ Wasserstoff oder Niederalkyl bedeutet; $R^2$ für Wasserstoff, Niederalkyl, Halogen oder Trifluormethyl steht; $R^3$ für Wasserstoff, Niederalkyl, Niederalkoxy, Halogen oder Trifluormethyl steht; $R^4$ für Wasserstoff, Niederalkyl, Hydroxymethyl, Carboxy, Niederalkoxycarbonyl, Carbamoyl oder N-Mono- oder N,N-Diniederalkylcarbamoyl steht; und pharmazeutisch annehmbare Salze davon.

Besonders bevorzugt sind die Verbindungen der Formel I, worin $R^1$ Wasserstoff oder Niederalkyl

bedeutet; $R^2$ für Wasserstoff oder Halogen steht; $R^3$ für Wasserstoff, Niederalkoxy oder Halogen steht; $R^4$ für Carboxy, Niederalkoxycarbonyl, Carbamoyl oder N-Mono-, oder N,N-Diniederalkylcarbamoyl steht; und pharmazeutisch annehmbare Salze davon.

Eine besonders bevorzugte Ausführungsform bezieht sich auf Verbindungen der Formel I, worin $R^1$ Wasserstoff oder Niederalkyl bedeutet; $R^2$ für Wasserstoff oder Halogen steht; $R^3$ für Wasserstoff, Niederalkoxy oder Halogen steht; $R^4$ für Niederalkoxycarbonyl oder Carbamoyl steht; und pharmazeutisch annehmbare Salze davon.

Am meisten bevorzugt sind die Verbindungen der Formel I, worin $R^1$ Niederalkyl bedeutet; $R^2$ und $R^3$ für Wasserstoff stehen; $R^4$ für Niederalkoxycarbonyl steht; und pharmazeutisch annehmbare Salze davon.

Im Zusammenhang mit der vorliegenden Anmeldung bezeichnet der Ausdruck "Nieder" Radikale mit bis zu und einschliesslich 7, vorzugsweise mit bis zu und einschliesslich 4 Kohlenstoffatomen. In Ausdrükken wie "Niederalkoxycarbonyl" bezieht sich der Ausdruck "Nieder" nur auf die Alkoxy-Einheit.

Niederalkyl ist vorzugsweise $C_1$-$C_4$-Alkyl, insbesondere Methyl oder Propyl. $C_3$-$C_7$-Alkenyl und $C_3$-$C_7$-Alkinyl, welches über ein gesättigtes Kohlenstoffatom gebunden ist, sind z.B. Allyl und Propargyl.

3 bis 7-gliedriges Cycloalkyl (wie in $R^1$) ist z.B. Cyclohexyl.

$C_2$-$C_7$-Alkyl, welches durch einen Substituenten ausgewählt aus der Gruppe bestehend aus Hydroxy, Amino, N-Mononiederalkylamino und N,N-Diniederalkylamino substituiert ist, wobei diese Substituenten vom Ringstickstoffatom durch mindestens 2 Kohlenstoffatome getrennt sind (wie in $R^1$) ist z.B. 2-(Hydroxy, Amino, Methylamino oder Dimethylamino)-ethyl.

Niederalkyl, welches durch einen Substituenten ausgewählt aus der Gruppe bestehend aus 3 bis 7-gliedrigem Cycloalkyl, Carboxy, Niederalkoxycarbonyl, Carbamoyl, N-Mononiederalkylcarbamoyl, N,N-Diniederalkylcarbamoyl und Niederalkanoyl substituiert ist (wie in $R^1$) ist z.B (Cyclohexyl, Carboxy, Methoxycarbonyl, Carbamoyl, N-Methylcarbamoyl, N,N-Dimethylcarbamoyl oder Acetyl)-methyl.

Niederalkyl, welches durch Phenyl oder Benzoyl substituiert ist, wobei jedes dieser Phenyl- oder Benzoylradikale unsubstituiert oder durch bis zu 3 Substituenten ausgewählt aus der Gruppe bestehend aus Niederalkyl, Niederalkoxy, Niederalkylthio, Halogen and Trifluormethyl substituiert ist (wie in $R^1$), ist z.B. Benzyl, Benzoylmethyl, p-Methylbenzyl, p-Methoxybenzyl, p-Methylthiobenzyl, p-Chlorbenzyl, p-Trifluormethylbenzyl, (p-Methylbenzoyl)methyl, (p-Methoxybenzoyl)methyl, (p-Methylthiobenzoyl)methyl, (p-Chlorbenzoyl)methyl oder (p-Trifluormethylbenzoyl)methyl.

Niederalkyl (wie in $R^2$, $R^3$ oder $R^4$) ist z.B. Methyl. Niederalkoxy (wie in $R^2$ oder $R^3$) ist z.B. Methoxy.

Halogen (wie in $R^2$ oder $R^3$) ist z.B. Brom oder Iod, bevorzugt aber Chlor.

Hydroxyniederalkyl (wie in $R^4$) ist vorzugsweise Hydroxymethyl. Niederalkoxycarbonyl (wie in $R^4$) ist vorzugsweise Methoxycarbonyl. N-Mononiederalkylcarbamoyl (wie in $R^4$) ist vorzugsweise N-Methylcarbamoyl, N,N-Diniederalkylcarbamoyl (wie in $R^4$) ist vorzugsweise N,N-Dimethylcarbamoyl.

Die Verbindungen der Formel I können Säureadditionssalze bilden, vorzugsweise pharmazeutisch annehmbare Säureadditionssalze, z.B. Salze von anorganischen oder organischen Säuren, z.B. starken Mineralsäuren, z.B. Halogenwasserstoffsäuren, z.B. Chlorwasserstoff oder Bromwasserstoff; Schwefel-, Phosphor- oder Salpetersäure, aliphatischen oder aromatischen Carbonsäuren oder Sulfonsäuren, z.B. Ameisen-, Essig-, Propion-, Bernstein-, Glycol-, Milch-, Apfel-, Wein-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminosalicyl-, Glucon-, Pamoa-, Nicotin-, Methansulfon-, Ethansulfon-, Hydroxyethansulfon-, Benzolsulfon-, p-Toluolsulfon-, Naphthalinsulfon-, Sulfanil-, Cyclohexylsulfamin- oder Ascorbinsäure; oder Aminosäuren, wie Arginin und Lysin.

Saure Verbindungen der Formel I, z.B. solche worin $R^4$ für Carboxy oder worin $R^1$ für Carboxyniederalkyl steht, können Metall- oder Ammoniumsalze bilden, vorzugsweise pharmazeutisch annehmbare und nicht toxische Metall- oder Ammoniumsalze, z.B. Alkalimetall- oder Erdalkalimetallsalze, z.B. Natrium-, Kalium-, Magnesium- oder Calciumsalze, oder Salze mit Ammoniak oder geeigneten organischen Aminen - aliphatischen, cycloaliphatischen, cycloaliphatisch-aliphatischen oder araliphatischen primären, sekundären oder tertiären Mono-, Di- oder Poly-aminen und auch heterocyclischen Basen, die für Salzbildung besonders geeignet sind - wie Niederalkylamine, z.B. Triethylamin, Hydroxyniederalkylamino, z.B. 2-Hydroxyethylamin, Bis(2-hydroxyethyl)amin, 2-Hydroxyethyldiethylamin oder Tris(2-hydroxyethyl)amin, basische aliphatische Ester von Carbonsäuren, z.B. 4-Aminobenzoesäure, 2-Diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, und auch Basen vom Pyridintyp, z.B. Pyridin, Collidin oder Chinolin. Verbindungen der Formel I mit einer freien Carboxygruppe können auch in Form von inneren Salzen vorliegen, d.h. in zwitterionischer Form.

Verbindungen der Formel I, die mehr als eine basische und/oder saure Gruppe haben, können Poly-Salze bilden, oder ein Teil des Moleküls kann in Form eines inneren Salzes vorliegen und ein anderer Teil in Form eines normalen Salzes.

Beispiele für Verbindungen, die mehr als eine basische Gruppe aufweisen, sind Verbindungen der Formel I, worin $R^1$ $C_2$-$C_7$-Alkyl bedeutet, welches durch Amino, N-Mononiederalkylamino oder N,N-Diniederalkylamino substituiert ist.

Zum Zwecke der Isolierung oder Reinigung ist es auch möglich, pharmazeutisch nicht annehmbare Salze zu verwenden. Für therapeutische Zwecke werden jedoch nur die pharmazeutisch annehmbaren nicht-toxischen Salze verwendet, und diese sind daher bevorzugt.

Die erfindungsgemässen Verbindung sind insbesondere als Serotonin-2-Rezeptor-Antagonisten in Säugetieren wirksam und als therapeutische Wirkstoffe zur Behandlung von Krankheiten und Zuständen, die auf die Wirkung eines Serotonin-2-Rezeptur-Antagonisten ansprechen, inklusive Krankheiten des zentralen Nervensystem, des cardio-vaskulären Systems und des gastrointestinalen Systems.

Die genannten Eigenschaften lassen sich in in-vitro und in-vivo Versuchen, vorzugsweise bei Säugetieren, z.B. Ratten, Hunden oder Affen, aber auch an entnommenen isolierten Organen, Gewebsproben oder enzymatischen Aufbereitungen nachweisen. Die Verbindungen können in-vitro in Form von Lösungen, z.B. wässrigen Lösungen, und in-vivo entweder enteral oder parenteral, mit Vorteil oral oder intravenös, z.B. in Gelatinekapseln, als Stärkesuspensionen oder in wässrigen Lösungen, appliziert werden. Der Dosisbereich in-vitro kann zwischen $10^{-6}$ und $10^{-9}$ molaren Konzentrationen betragen. Der Dosisbereich in-vivo kann zwischen etwa 0,10 und 30 mg/kg/Tag, vorzugsweise zwischen etwa 0,50 und 20 mg/kg/Tag, und insbesondere zwischen etwa 1,0 und 10 mg/kg/Tag betragen.

Die vorstehend beschriebenen Verbindungen sind z.B. in den folgenden Testsystemen, welche auf Serotonin-2-Rezeptor-Antagonismus hinweisen, wirksam:

Die Serotonin-2-Rezeptor-Bindungseigenschaften (dieser Rezeptor wird auch 5-Hydroxytryptamin-2- oder 5HT-2-Rezeptor genannt) werden in vitro bestimmt, indem die Fähigkeit dieser Verbindungen gemessen wird, die spezifische Bindung von $^3$H-Ketanserin zu inhibieren. Diese Messungen werden in Membran-Präparationen des frontalen/parietalen Cortex von männlichen Sprague-Dawley Ratten vorgenommen, grundsätzlich wie von Battaglia et al. in Life Sciences 33, 2011 (1983) beschrieben. $IC_{50}$-Werte, die die Konzentrationen einer Verbindung wiedergeben, die benötigt sind, um 50 % des $^3$H-Ketanserin zu verdrängen, werden durch Log-logit-Analyse spezifischer Bindungsdaten bestimmt.

Repräsentative erfindungsgemässe Verbindungen sind in dem Serotonin-2-Rezeptor Bindungstest mit einem $IC_{50}$-Wert von etwa 4nM wirksam.

Die Selektivität für den 5HT-2 Rezeptor kann bestimmt werden, indem auch die spezifische Bindung an Serotonin-1 (5HT-1)-Rezeptoren gemessen wird, z.B. gemäss D.N. Middlemiss und J.R. Fozard in Eur. J. Pharmacol. 90, 151 (1980).

In vivo verdrängen die erfindungsgemässen Verbindungen die Bindung von $^3$H-Spiperon an Serotonin-2-Rezeptoren im Rattenhirn. Repräsentative erfindungsgemässe Verbindungen verdrängen die Bindung von $^3$H-Spiperon mit einem $ED_{50}$-Wert von etwa 2 mg/kg i.p.

Der Serotonin-2-Antagonismus bzw. die Blockade lässt sich in vivo durch Messen der Inhibierung von Kopfzuckungen nachweisen, welche an der Ratte durch 5-Hydroxytryptophan (den metabolitischen Vorläufer von Serotonin) induziert werden. Dieser Test, bei dem im zentralen Nervensystem Serotonin-2-Rezeptor-Antagonismus an der Ratte festgestellt wird, ist in Neuropharmacology 16, 663 (1977) und in J. Pharmacol. Exp. Ther. 228, 133 (1984) beschrieben. Der Test wird wie folgt ausgeführt:

Man lässt männliche Wistar-Ratten (120-180 g) 18 Stunden vor dem Test hungern, gibt ihnen aber Wasser nach Belieben. Alle Tiere werden mit dem peripheren Decarboxylase-Inhibitor Alpha-methyl-DOPA-hydrazin (Carbidopa, 25 mg/kg i.p., 4,0 ml/kg) vorbehandelt, gefolgt 30 Minuten später von 5-Hydroxytryptophan (5-HTP, 100 mg/kg s.c., 4,0 ml/kg). 90 Minuten nach Erhalt von 5-HTP werden die Ratten einzeln in Plexiglasbeobachtungskäfige gesetzt, und die Häufigkeit von Kopfzuckungen jedes Tieres wird während einer 10-minütigen Beobachtungsperiode gezählt. Die Test-Verbindung oder der Träger werden entweder nach 0,5 Stunden in einer Dosis von 1,0 ml/kg i.p. verabreicht oder nach 1,2 oder 4 Stunden in einer Dosis von 10 ml/kg p.o. vor der Beobachtungsperiode. $ED_{50}$-Werte werden gemäss Probit-Analyse gestimmt.

Repräsentative erfindungsgemässe Verbindungen sind in dem Kopfzuckungstest bei einer Dosis von etwa 3 mg/kg p.o. in der Ratte wirksam.

Weitere biologische Wirkungen der erfindungsgemässen Verbindungen die auf den die Serotonin-2-Rezeptoren blockierenden Eigenschaften der Verbindungen beruhen, z.B. Wirkungen auf das zentrale Nervensystem und auf das cardio-vaskuläre System, können unter Verwendung von bekannten Tiertests bestimmt werden. Z.B. können Wirkungen, die anxiolytische Eigenschaften anzeigen, gemäss dem Standard Cook-Davidson Konflikt Modell an der Ratte festgestellt werden. Ein Häufigerwerden von Bestrafungen nach sich ziehenden Verhaltensweisen zeigt hierbei eine anxiolytische Wirkung an. Antihypertensive Eigenschaften können an der spontan hypertensiven Ratte gezeigt werden. Antithrombotische Effekte können durch die Inhibierung von Serotonin-induzierter Plättchen-Aggregationen gezeigt werden.

Daher besitzen die erfindungsgemässen Verbindung wertvolle Eigenschaften in Säugern, insbesondere als Serotonin-2-Rezeptor-Antagonisten, zur Behandlung von Krankheiten des zentralen Nervensystems, wie Angstzuständen, Depressionen und Manien, zur Behandlung von gastrointestinalen Krankheiten, wie Geschwüren, und zur Behandlung von cardio-vaskulären Krankheiten, wie Bluthochdruck und Thrombosen.

Die erfindungsgemässen Verbindungen, die serotonergische Funktionen an zentralen Serotonin-2-Rezeptoren inhibieren, werden insbesondere als nützlich angesehen als anxiolytische Wirkstoffe zur Behandlung von Angstzuständen, insbesondere da sie bei wirksamen anxiolytischen Dosen geringe oder keine Sedation oder Leistungsbeeinträchtigung hervorrufen.

Die Verbindungen der Formel I, ihre 2-N-Oxide und Salze davon werden gemäss den folgenden Verfahren hergestellt:

a) eine Verbindung der Formel II,

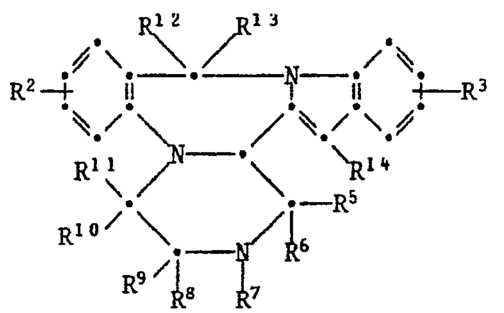

(II)

worin $R^2$ und $R^3$ die vorstehend angegebenen Bedeutungen aufweisen; $R^5$ und $R^6$ Wasserstoff bedeuten oder $R^5$ und $R^6$ zusammen Oxo bedeuten; $R^7$ die Bedeutung von $R^1$ hat und vorzugsweise für Wasserstoff, Niederalkyl, $C_2$-$C_7$-Alkyl, welches durch Hydroxy- Ami no oder Mono- oder Diniederalkylamino substituiert ist, $C_3$-$C_7$-Alkenyl, $C_3$-$C_7$-Alkinyl, oder Niederalkyl, welches durch 3 bis 7-gliedriges Cycloalkyl oder durch gegebenenfalls substituiertes Phenyl substituiert ist, steht; $R^8$ und $R^9$ Wasserstoff bedeuten oder $R^8$ und $R^9$ zusammen Oxo bedeuten; $R^{10}$ und $R^{11}$ Wasserstoff oder $R^{10}$ und $R^{11}$ zusammen Oxo bedeuten; $R^{12}$ und $R^{13}$ Wasserstoff oder $R^{12}$ und $R^{13}$ zusammen Oxo bedeuten; und $R^{14}$ die vorstehend für $R^4$ angegebenen Bedeutungen aufweist und vorzugsweise für Wasserstoff, Niederalkyl, das durch Hydroxy substituiert sein kann, oder Niederalkoxycarbonyl steht, mit der Massgabe, dass zumindest eine Oxo-Gruppe in Form von $R^5$ und $R^6$, $R^8$ und $R^9$, $R^{10}$ und $R^{11}$ oder $R^{12}$ und $R^{13}$ anwesend ist; wird mit einem geeigneten Reduktionsmittel behandelt, das in der Lage ist, besagte Oxo-Gruppe(n) in (eine) Methylen-Gruppe(n) umzuwandeln; oder

b) eine Verbindung der Formel III

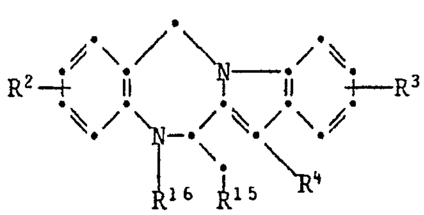

(III)

worin $R^{15}$ eine nukleophile Abgangsgruppe X bedeutet, $R^{16}$ die Gruppe $-CH_2$-$CH_2$-$NHR^7$ bedeutet, worin $R^7$ für Wasserstoff, Niederalkyl, $C_2$-$C_7$-Alkyl, welches durch Hydroxy, das durch eine Hydroxyschutzgruppe geschützt sein kann, substituiert ist, $C_2$-$C_7$-Alkyl, welches durch Amino oder Mononiederalkylamino, von denen jede Gruppe durch eine Aminoschutzgruppe geschützt ist, substituiert ist, $C_2$-$C_7$-Alkyl, welches durch Diniederalkylamino substituiert ist, steht, oder $R^7$ $C_3$-$C_7$-Alkenyl bedeutet, $C_3$-$C_7$-Alkinyl, oder 3 bis 7-gliedriges Cycloalkyl; oder eine Verbindung der Formel III, worin $R^{15}$ die Gruppe $-NHR^7$ bedeutet, worin $R^7$ wie vorstehend definiert ist und $R^{16}$ die Gruppe $-CH_2CH_2$-X bedeutet, worin X für eine nukleophile Abgangsgruppe steht; oder eine Verbindung der Formel III, worin $R^{15}$ die Gruppe -N-($R^7$)-$CH_2CH_2$-X bedeutet, worin die Substituenten die vorstehend erwähnten Bedeutungen aufweisen und $R^{16}$ Wasserstoff bedeutet, oder ein reaktionsfähiges Derivat davon; und die anderen Substituenten die vorstehend angegebenen Bedeutungen aufweisen; wird intramolekular cyclisiert, und anwesende Schutzgruppen werden entfernt; oder
c) eine Verbindung der Formel IV

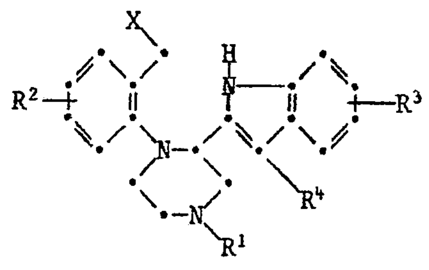

(IV)

worin X eine nukleophile Abgangsgruppe bedeutet und die anderen Substituenten die vorstehend angegebenen Bedeutungen aufweisen, oder ein reaktionsfähiges Derivat davon, wird intramolekular cyclisiert; und, wenn erwünscht, eine Verbindung, die nach einem der Verfahren a) bis c) erhalten wurde, wird in eine andere erfindungsgemässe Verbindung übergeführt, z.B. durch Veresterung, Amidierung, Decarboxylierung oder Reduktion von freiem Carboxy $R^4$, oder durch Ueberführung von verestertem oder amidiertem Carboxy $R^4$ in freies Carboxy, oder durch Umwandlung einer Verbindung der Formel I, worin $R^1$ Wasserstoff bedeutet, in eine Verbindung der Formel I, worin $R^1$ die vorstehenden Bedeutungen ausser Wasserstoff aufweist, oder durch Umwandeln einer Verbindung der Formel I, worin $R^1$ die vorstehend angegebenen Bedeutungen aufweist ausser Wasserstoff, in das 2-N-Oxid, oder durch Umwandeln des 2-N-Oxides einer Verbindung der Formel I in die entsprechende Verbindung der Formel I, und/oder ein erhaltenes Salz wird in die freie Verbindung oder in ein anderes Salz übergeführt und/oder eine erhaltene freie Verbindung, die eine salzbildende Gruppe aufweist, wird in ein Salz übergeführt.

Die vorstehend erwähnten Verfahren und zusätzlichen Operationen werden nachstehend im einzelnen erläutert. Verfahren a) ist bevorzugt.

Verfahren a):

Ein geeignetes Reduktionsmittel ist vorzugsweise Diboran. Komplexe Hydride, wie Alan, Alkalimetallaluminiumhydride, z.B. Lithiumaluminiumhydrid, Natriumtritertiärbutoxyaluminiumhydrid oder Natriumbis(2-methoxyethoxy)aluminiumhydrid sind weitere Beispiele geeigneter Reduktionsmittel. Wenn $R^{14}$ für Niederalkoxy carbonyl oder Carbamoyl steht, reduzieren einige der vorstehend genannten Reduktionsmitel ausser Boran gleichzeitig die Niederalkoxycarbonyl-oder Carbamoyleinheit.

Bevorzugt sind solche Ausgangsverbindungen der Formel II, worin $R^5$ und $R^6$ zusammen und $R^{10}$ und $R^{11}$ zusammen jeweils Oxo bedeuten, und $R^8$, $R^9$, $R^{12}$ und $R^{13}$ für Wasserstoff stehen, wie sie durch Formel IIA wiedergegeben werden,

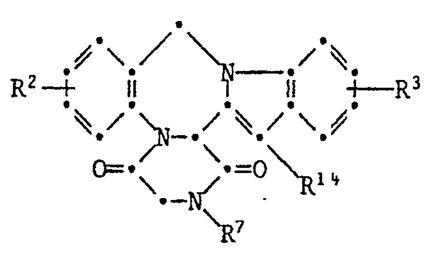

(IIA)

worin $R^7$ Wasserstoff, Niederalkyl, $C_3$-$C_7$-Alkenyl, welches über ein gesättigtes Kohlenstoffatom gebunden ist, $C_3$-$C_7$-Alkinyl, welches über ein gesättigtes Kohlenstoffatom gebunden ist, 3 bis 7-gliedriges Cycloalkyl, $C_2$-$C_7$-Alkyl, welches durch einen Substituenten ausgewählt aus der Gruppe bestehend aus Hydroxy, Amino, N-Mononideralkylamino und N,N-Diniederalkylamino substituiert ist, wobei diese Substituenten vom Ringstickstoffatom durch mindestens 2 Kohlenstoffatome getrennt sind, bedeutet; oder worin $R^7$ Niederalkyl, welches durch einen Substituenten substituiert ist, welcher ausgewählt ist aus der Gruppe bestehend aus 3 bis 7-gliedrigem Cycloalkyl, Niederalkoxycarbonyl, Carbamoyl, und Phenyl, welches unsubstituiert oder durch bis zu 3 Substituenten ausgewählt aus der Gruppe Niederalkyl, Niederalkoxy, Niederalkylthio,

Halogen und Trifluormethyl substituiert ist, bedeutet; $R^2$ und $R^3$ unabhängig voneinander Wasserstoff, Niederalkyl, Hydroxy, Niederalkoxy, Halogen oder Trifluormethyl bedeuten; und $R^{14}$ für Wasserstoff, Niederalkyl, Hydroxyniederalkyl, Niederalkoxycarbonyl oder Carbamoyl steht.

Bevorzugte Ausgangsverbindungen sind solche Verbindungen der Formel IIA, die in die bevorzugten Ausführungsformen der Erfindung übergeführt werden können, insbesondere die Verbindungen der Formel IIA, worin $R^7$ für Wasserstoff oder Niederalkyl steht, $R^2$ Wasserstoff, Niederalkyl, Halogen oder Trifluormethyl bedeutet, $R^3$ für Wasserstoff, Niederalkyl, Niederalkoxy, Halogen oder Trifluormethyl steht, und $R^{14}$ Wasserstoff, Niederalkyl oder Niederalkoxycarbonyl bedeutet.

Diese Ausgangsverbindungen der Formel IIA werden z.B. hergestellt, indem ein Niederalkylester, wie ein Ethylester der Formel V,

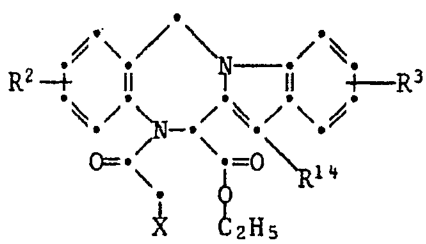

(V)

worin die Substituenten $R^2$, $R^3$ und $R^{14}$ die vorstehend angegebenen Bedeutungen aufweisen, X eine Abgangsgruppe bedeutet, wie Chlor, mit einem Amin der Formel $R^7$-$NH_2$, worin $R^7$ die vorstehend angegebenen Bedeutungen aufweist, mit der Massgabe, dass Amino, Niederalkylamino und, wenn notwendig, Hydroxygruppen, welche in $R^7$ anwesend sind, durch eine geeignete Amino- oder Hydroxyschutzgruppe geschützt sind und dass diese Schutzgruppen später entfernt werden, umgesetzt wird.

Diese Ausgangsverbindungen der Formel V werden hergestellt, indem z.B. eine Verbindung der Formel VI,

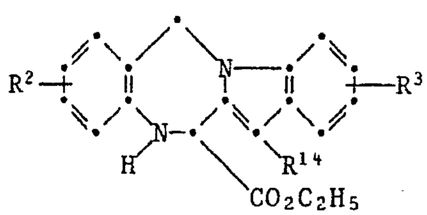

(VI)

worin die Substituenten die vorstehend angegebenen Bedeutungen aufweisen, mit der Massgabe, dass eine Hydroxygruppe, welche in $R^{14}$ anwesend ist, wenn gewünscht, durch eine Hydroxyschutzgruppe geschützt ist, mit z.B. Chloressigsäurechlorid der Formel $Cl$-$CH_2$-$CO$-$Cl$ umgesetzt wird, wobei, wenn eine Schutzgruppe anwesend ist, diese später entfernt wird.

Die Ausgangsverbindungen der Formel VI werden z.B. durch Reduktion einer Verbindung der Formel VII hergestellt

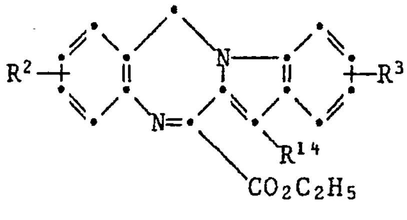

(VII)

worin die Substituenten die vorstehend angegebenen Bedeutungen aufweisen, mit z.B. Natriumhypophosphit ($NaH_2PO_2$) in Anwesenheit von 5 % Palladium auf Kohle.

Die Ausgangsverbindungen der Formel VIII werden z.B. durch intramolekulare Cyclisierung hergestellt, z.B. von einer Verbindung der Formel VIII,

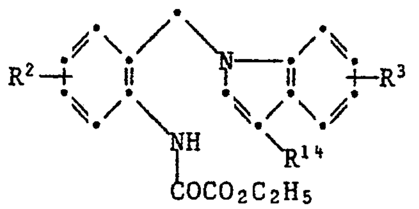

(VIII)

worin die Substituenten die vorstehend angegebenen Bedeutungen aufweisen, mit Hilfe von z.B. Phosphoroxychlorid und Phosphorpentoxid. Vorzugsweise wird die Umsetzung unter Verwendung eines Ausgangsmaterials der Formel VIII ausgeführt, worin $R^{14}$ Niederalkoxycarbonyl bedeutet. Dieser Niederalkoxycarbonyl-Substituent $R^{14}$ kann in einer späteren Stufe in andere Substituenten, z.B. Carboxy, Hydroxymethyl usw. übergeführt werden.

Die Ausgangsverbindungen der Formel VIII werden z.B. durch Umsetzung einer Verbindung der Formel IX,

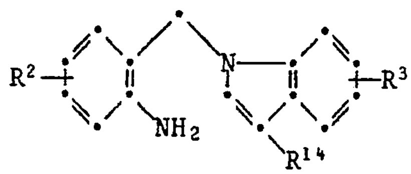

(IX)

mit z.B. Ethyloxalylchlorid in Gegenwart einer Base wie Pyridin hergestellt.

Die Ausgangsverbindungen der Formel IX wiederum werden durch Reduktion der Nitrogruppe in einer entsprechenden nitrosubstituierten Verbindung, beispielsweise mit Eisen in Essigsäure, hergestellt. Die durch Nitro substituierten Verbindungen werden durch Behandeln einer Verbindung der Formel X

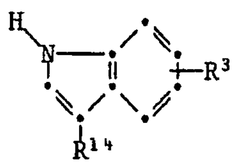

(X)

mit z.B. einem durch $R^2$ substituierten o-Nitrobenzylhalogenid in Gegenwart einer starken Base, z.B. von Lithiumamid, hergestellt. Die Ausgangsverbindungen der Formel X, z.B. Indol-3-carbonsäureester, sind bekannt.

Statt dessen kann auch eine Verbindung der Formel IX zuerst mit z.B. Chloracetylchlorid behandelt werden, so dass nach Cyclisierung mit Phosphoroxychlorid/Phosphorpentoxid und Reduktion der C-N-Doppelbindung eine Verbindung der Formel VIa erhalten wird:

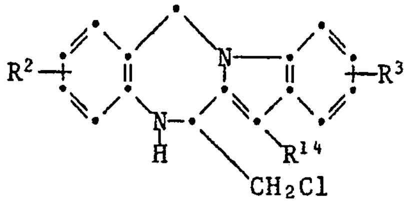

(VIa)

Kondensation mit beispielsweise Choracetylchlorid, gefolgt von Behandlung mit einer Verbindung der Formel $R^7$-$NH_2$, geeigneterweise geschützt, wenn notwendig, ergibt ein Zwischenprodukt der Formel II, worin $R^{10}$ und $R^{11}$ zusammen für Oxo stehen, $R^5$, $R^6$, $R^8$, $R^9$, $R^{12}$ und $R^{13}$ Wasserstoff bedeuten, und $R^2$, $R^3$, $R^7$ und $R^{14}$ die vorstehend angegebenen Bedeutungen aufweisen.

Statt dessen kann eine Verbindung der Formel VIa z.B. auch mit Ethyloxalylchlorid umgesetzt werden, erfolgt von Behandlung mit einer Verbindung der Formel $R^7$-$NH_2$, geeigneterweise geschützt, wenn

erforderlich, so dass eine Verbindung der Formel II erhalten wird, worin $R^8$ und $R^9$ zusammen und $R^{10}$ und $R^{11}$ zusammen für Oxo stehen, $R^5$, $R^6$, $R^{12}$ und $R^{13}$ Wasserstoff bedeuten, und $R^2$, $R^3$, $R^7$ und $R^{14}$ die vorstehend angegebenen Bedeutungen aufweisen.

Statt dessen kann auch eine Verbindung der Formel VIa mit einem geeigneterweise $\alpha$-substituierten Essigsäureester umgesetzt werden, z.B. mit Bromessigsäureethylester, gefolgt von Behandlung mit einer Verbindung der Formel $R^7$-$NH_2$, geeigneterweise geschützt, wenn erforderlich, so dass eine Verbindung der Formel II erhalten wird, worin $R^8$ und $R^9$ zusammen für Oxo stehen und $R^5$, $R^6$, $R^{10}$, $R^{11}$, $R^{12}$ und $R^{13}$ Wasserstoff bedeuten; und $R^2$, $R^3$, $R^7$ und $R^{14}$ die vorstehend angegebenen Bedeutungen aufweisen.

Verfahren b):

Die Cyclisierung gemäss Verfahren b) kann ausgeführt werden, indem eine Lösung einer Verbindung der Formel III, worin die Abgangsgruppe X z.B. Chlor bedeutet, in einem nicht-wässrigen polaren Lösungsmittel, vorzugsweise in Anwesenheit einer anorganischen Base, wie Kaliumcarbonat, oder eines tertiären Amins, wie Triethylamin, erhitzt wird.

Die Ausgangsverbindungen der Formel III, z.B. worin $R^{15}$ eine Abgangsgruppe wie Chlor bedeutet und $R^{16}$ für die Gruppe $-CH_2-CH_2-NHR^7$ steht, können hergestellt werden, indem eine Verbindung der Formel VIa mit z.B. 1-Brom-2-chlorethan kondensiert wird, in Gegenwart einer starken Base wie Natriumhydrid in einem polaren Lösungsmittel wie Dimethylformamid, so dass eine Verbindung der Formel III erhalten wird, worin z.B. $R^{15}$ Chlor bedeutet und $R^{16}$ für $-CH_2-CH_2-Cl$ steht, und indem danach diese Verbindung mit einem Amin der Formel $R^7$-$NH_2$ unter Bedingungen einer Aminoalkylierung umgesetzt wird, in einem inerten Lösungsmittel, vorzugsweise in Gegenwart einer Base wie Triethylamin oder Kaliumcarbonat und gegebenenfalls eines Iodsalzes, wie Kaliumiodid.

Verfahren c):

Die Cyclisierung gemäss Verfahren c) kann durch Behandeln einer Verbindung der Formel IV mit einer starken wasserfreien Base, wie Lithiumamid, durchgeführt werden.

Im Zusammenhang mit den vorstehend erwähnten Verfahren bedeutet eine Abgangsgruppe vorzugsweise eine reaktionsfähige veresterte Hydroxygruppe, wie insbesondere Halogen, z.B. Chlor, Brom oder Iod oder aliphatisch oder aromatisch substituiertes Sulfonyloxy, z.B. Methylsulfonyloxy oder 4-Methylphenylsulfonyloxy (Tosyloxy).

Die Verbindungen der Formel I oder Ausgangsverbindungen, die zu diesen umgesetzt werden können, können in andere Verbindungen der Formel I oder entsprechende Ausgangsverbindungen übergeführt werden, unter Verwendung von chemischen Verfahren, die im Stand der Technik bekannt sind und in dieser Anmeldung erläutert werden.

Erfindungsgemässe Verbindungen, z.B. solche der Formel I, worin der Stickstoffsubstituent $R^1$ Wasserstoff bedeutet, können in Verbindungen übergeführt werden, worin $R^1$ einen anderen Substituenten als Wasserstoff bedeutet, z.B. a) durch Umsetzung mit einem reaktionsfähigen veresterten Derivat, z.B. einem Halogenid, welches der Gruppe $R^1$ entspricht, oder b) durch Umsetzung mit einem Aldehyd z.B. einem Niederalkylcarboxaldehyd, in Gegenwart eines Reduktionsmittels wie Natriumcyanborhydrid, so dass eine Verbindung der Formel I erhalten wird, in der $R^1$ für Niederalkyl steht, oder c) durch reduktive Alkylierung mit Formaldehyd und Ameisensäure, so dass eine Verbindung erhalten wird, in der $R^1$ für Methyl steht.

Erfindungsgemässe Verbindungen, z.B. solche der Formel I, worin $R^1$ für Methyl steht, insbesondere solche, worin $R^4$ Wasserstoff oder Niederalkyl bedeutet, können auch hergestellt werden, indem die entsprechenden Verbindungen, worin $R^1$ Wasserstoff bedeutet, mit einem Halogenameisensäure-Niederalkyl- oder -phenylniederalkylester, wie Chlorameisensäureethylester, umgesetzt werden, so dass zuerst Verbindungen, worin $R^1$ z.B. Alkoxycarbonyl oder Phenylalkoxycarbonyl ist, erhalten werden, und dieses Acylderivat mit einem einfachen oder komplexen Leichtmetallhydrid wie Lithiumaluminiumhydrid, Natriumtritertiärbutoxy- oder Bis(2-methoxyethoxy)aluminiumhydrid reduziert wird.

Verbindungen, in denen der Stickstoffsubstituent Benzyl oder gegebenenfalls substituiertes Benzyl ist, können zu den entsprechenden Verbindungen hydrogenolysiert werden, in denen der Stickstoffsubstituent Wasserstoff ist, z.B. mit Wasserstoff in Gegenwart eines Hydrogenolysekatalysators, z.B. Palladium auf Kohle.

Ungesättigte N-substituierte Verbindungen, wie solche, die einen Alkenyl- oder Alkinylsubstituenten tragen, können auch mit katalytisch aktiviertem Wasserstoff hydriert werden, so dass Verbindungen erhalten werden, die den entsprechenden N-Alkyl Substituenten tragen.

Verbindungen, die an den aromatischen Ringen durch Hydroxy substituiert sind (Phenole), können in

die Verbindungen übergeführt werden, die durch Niederalkoxy substituiert sind, wobei Verfahren verwendet werden, die für die Alkylierung von Phenolen bekannt sind, so kann z.B. eine Verbindung der Formel I, worin $R^2$ und/oder $R^3$ Hydroxy bedeuten, in Gegenwart einer Base, gegebenenfalls unter Bedingungen der Phasentransferkatalyse, mit einem reaktionsfähigen veresterten Derivat eines Niederalkanols, z.B. einem Halogenderivat davon, umgesetzt werden.

Die Umwandlung von Verbindungen, worin $R^2$ und/oder $R^3$ Niederalkoxy bedeutet, in Verbindungen, worin $R^2$ und/oder $R^3$ Hydroxy ist, wird auf bekannte Weise durchgeführt, z.B. mit einer Mineralsäure, wie Iodwasserstoffsäure, oder, vorzugsweise für Verbindungen, worin Niederalkoxy Methoxy ist, mit z.B. Bortribromid in Dichlormethan, mit Natruim- oder oder Lithiumdiphenylphosphid in Tetrahydrofuran, oder durch Erhitzen mit Pyridinhydrochlorid.

Erfindungsgemässe Verbindungen und entsprechende Ausgangsverbindungen, z.B. die Verbindungen der Formeln I oder II, worin z.B. der Substituent $R^4$ oder $R^{14}$ für Niederalkoxycarbonyl steht, können durch Hydrolyse der Ester, z.B. mit wässriger Base, wie Natriumhyd roxid-oder Kaliumhydroxidlösungen, in die entsprechenden Carbonsäuren übergeführt werden.

Erfindungsgemässen Verbindungen und entsprechende Ausgangsverbindungen, z.B. die Verbindungen der Formeln I oder II, worin $R^4$ oder $R^{14}$ für Carboxy stehen, können in die entsprechenden Verbindungen übergeführt werden, worin $R^4$ oder $R^{14}$ Wasserstoff bedeutet, indem ein Decarboxylierungsverfahren angewandt wird, z.B. vorzugsweise eine Behandlung mit einer Lösung einer starken Mineralsäure, wie Chlorwasserstoffsäure, bei erhöhter Temperatur, vorzugsweise für einige Stunden.

Besagte Carbonsäuren können auch verestert werden z.B. zu Verbindungen der Formeln I oder II, worin $R^4$ oder $R^{14}$ Niederalkoxycarbonyl bedeuten, nach bekannten Veresterungsverfahren, z.B. durch Behandlung eines funktionellen Derivats, wie eines Acylhalogenids oder eines gemischten Anhydrids, z.B. eines Abkömmlings eines Niederalkylhalogencarbonats, wie von Chlorameisensäureethylester, mit dem geeigneten Alkohol.

Erfindungsgemässe Verbindungen und entsprechende Ausgangsverbindungen, z.B. die Verbindungen der Formeln I und II, worin $R^4$ oder $R^{14}$ Niederalkoxycarbonyl bedeuten, können in Verbindungen übergeführt werden, worin $R^4$ oder $R^{14}$ für Carbamoyl, N-Mono-oder N,N-Diniederalkylcarbamoyl stehen, durch Behandlung mit wasserfreiem Ammoniak oder den entsprechenden N-Mono- oder N,N-Diniederalkylaminen in einem polaren Lösungsmittel wie Tetrahydrofuran.

Erfindungsgemässe Verbindungen und entsprechende Ausgangsverbindungen, z.B. die Verbindungen der Formeln I oder II, worin $R^4$ oder $R^{14}$ Formyl bedeuten, können aus den entsprechenden Verbindungen der Formeln I oder II hergestellt werden worin $R^4$ oder $R^{14}$ Wasserstoff bedeuten, durch Behandlung mit Dimethylformamid in Gegenwart von Phosphoroxychlorid. Verbindungen, worin $R^4$ Carboxy bedeutet, können durch Oxidation solcher Verbindungen erhalten werden.

Erfindungsgemässe Verbindungen, z.B. die Verbindungen der Formel I, worin $R^4$ für Hydroxymethyl steht, können durch Reduktion entsprechender Verbindungen der Formel I, worin $R^4$ für Niederalkoxycarbonyl steht, mit einem Reduktionsmittel wie Lithiumaluminiumhydrid oder Natriumbis(2-methoxyethoxy)-aluminiumhydrid hergestellt werden. Verbindungen, worin $R^4$ für Carboxy steht, können durch Oxidation solcher Verbindungen hergestellt werden.

Erfindungsgemässe Verbindungen, z.B. die Verbindungen der Formel I, worin $R^4$ Methyl bedeutet, können durch Behandlung einer entsprechenden Verbindung, worin $R^4$ Formyl bedeutet, mit Diboran in Tetrahydrofuran hergestellt werden.

Tertiäre Amine der Formel I (worin $R^1$ nicht Wasserstoff bedeutet) können in die entsprechenden 2-N-Oxide übergeführt werden, z.B. mit organischen Persäuren, wie niederen Alkanpercarbonsäure oder Perbenzoesäuren, z.B. m-Chlorperbenzoesäure, vorzugsweise bei Temperaturen in der Nähe von oder unterhalb von Raumtemperatur.

In Ausgangsverbindungen, die in erfindungsgemässe Verbindungen übergeführt werden, wie hierin beschrieben, sind funktionelle Gruppen, wie Carbonyl (Formyl oder Keto), Carboxy, Amino und Hydroxy und Mercapto, gegebenenfalls durch konventionelle Schutzgruppen, die üblicherweise in der organischen Chemie angewendet werden, geschützt. Geschütztes Carbonyl, Carboxy, Amino, Hydroxy und Mercapto sind solche Gruppen, die unter milden Bedingungen in freies Carbonyl, Carboxy, Amino, Hydroxy oder Mercapto übergeführt werden können, ohne dass das molekulare Gerüst zerstört wird oder dass andere unerwünschte Nebenreaktionen ablaufen.

Der Grund, aus dem Schutzgruppen eingeführt werden, besteht darin, dass funktionelle Gruppen unter den Reaktionsbedingungen, die zur Durchführung einer erwünschten chemischen Umwandlung angewendet werden, keine unerwünschten Reaktionen mit den Reaktionskomponenten eingehen. Die Notwendigkeit und die Auswahl von Schutzgruppen für eine bestimmte Reaktion sind dem Fachmann g eläufig und hängen von der Natur der funktionellen Gruppe, die geschützt werden soll, von der Struktur und Stabilität des

Moleküls, welches den Substituenten trägt, und von den Reaktionsbedigungen ab.

Bekannte Schutzgruppen, die diese Bedingungen erfüllen, sowie ihre Einführung und Abspaltung sind beispielsweise beschrieben in J.F.W. McOmie, "Protective Groups in Organic Chemistry", Plenum Press, London, New York 1973, T.W. Greene, "Protective Groups in Organic Synthesis", Wiley, New York 1984, und ebenso in "The Peptides", Vol. I, Schröder und Lübke, Academic Press, London, New York 1965, wie auch in Houben-Weyl, "Methoden der Organischen Chemie", Vol. 15/1, Georg Thieme Verlag, Stuttgart, 1974.

Beispielsweise kann ein basisches primäres oder sekundäres Amin in Form von leicht spaltbaren Amiden geschützt sein, z.B. als Acylderivat wie als Benzyloxycarbonyl- (Carbobenzyloxy) oder als Tertiärbutyloxycarbonylderivat, oder als eine andere leicht abspaltbare N-Schutzgruppe.

Eine Carboxygruppe kann in Form eines leicht spaltbaren Esters geschützt sein, z.B. als Benzylester, als Tertiärbutylester usw. wie üblicherweise verwendet.

Eine Hydroxygruppe kann in Form von Estern, z.B. als Acylderivat, z.B. als Niederalkanoyl-, Benzyloxycarbonyl- oder Niederalkoxycarbonylester, oder in Form von Ethern, z.B. als Tetrahydropyranyl- oder Benzylether, geschützt werden.

In einer erhaltenen geschützten Verbindung der Formel I oder in einer Ausgangsverbindungen, worin eine oder mehrere funktionelle Gruppen geschützt sind, können die geschützten funktionellen Gruppen nach an sich bekannten Methoden, z.B. durch Solvolyse, insbesondere Hydrolyse mit einer Säure, oder durch Reduktion, insbesondere Hydrogenolyse, freigesetzt werden.

Die oben genannten nachträglichen Reaktionen werden nach an sich bekannten Methoden, in Gegenwart oder Abwesenheit von Verdünnungsmitteln, vorzugsweise solchen, welche gegenüber den Reagenzien inert sind und diese lösen, Katalysatoren, Kondensations- oder anderen oben genannten Mitteln, und/oder in einer inerten Atmosphäre, unter Kühlung, bei Raumtemperatur oder bei erhöhter Temperatur, vorzugsweise beim Siedepunkt der verwendeten Lösungsmittel, bei normalem oder erhöhtem Druck, durchgeführt.

Die Erfindung betrifft ebenfalls Abänderungen der vorliegenden Verfahren, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, z.B. wie oben und in den Beispielen illustriert.

In den genannten Reaktionen werden vorteilhafterweise solche Ausgangsstoffe verwendet, welche zu den weiter vorn als bevorzugt beschriebenen Verbindungen führen.

Die Erfindung betrifft auch neue Ausgangsstoffe und Verfahren zu ihrer Herstellung.

Das Kohlenstoffatom in Position 16b der erfindungsgemässen Verbindungen ist ein asymmetrisches Kohlenstoffatom. Diese Verbindungen können daher entweder als Racemate oder als optische Isomere (Antipoden) vorliegen. Ferner können die Verbindungen je nach der Art der vorhandenen Substituenten zusätzlich in Form geometrischer Isomere oder als Mischungen von Racematen oder optischen Antipoden (Diastereoisomerengemische) vorliegen. Sämtliche vorstehend aufgeführten Isomere fallen unter den Umfang dieser Erfindung.

Erhaltene geometrische oder diastereomere Isomerengemische der obigen Verbindungen oder Zwischenprodukte können nach an sich bekannten Methoden in die einzelnen racemischen oder optisch aktiven Isomeren getre nnt werden, z.B. durch fraktionierte Destillation. Kristallisation und/oder Chromatographie. Racemische Produkte der Formel I können gleichfalls in die optischen Antipoden gespalten werden, z.B. durch fraktionierte Kristallisation von d- und ℓ-(Tartraten, Dibenzoyltartraten, Mandelaten, Camphersulfaten) von Verbindungen, die eine basische, salzbildende Gruppe haben, oder von d- und ℓ-(α-Methylbenzylamid, Cinchonidin, Cinchonin, Chinin, Chinidin, Ephedrin, Dehydroabietylamin, Brucin oder Strychnin)-Salzen von Verbindungen, die eine saure, salzbildende Gruppe besitzen.

Bevorzugt wird jeweils das wirksamere Isomere und/oder der wirksamere Antipode der erfindungsgemässen Verbindungen isoliert.

Die Verbindungen der vorliegenden Erfindung werden entweder in freier oder in Form ihrer Salze erhalten. Jede erhaltene Base kann in das entsprechende Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metall- oder Ammoniumhydroxids oder eines basischen Salzes, z.B. eines Alkalimetallhydroxids oder -carbonats, oder eines Kationenaustauschers.

Infolge der engen Beziehung zwischen den freien Verbindungen und ihren Salzen sind in diesem Zusammenhang unter freien Verbindungen sinn- und zweckgemäss gegebenenfalls immer auch die

entsprechenden Salze zu verstehen.

Die Verbindungen der Erfindung und ihre Salze können auch in Form ihrer Hydrate erhalten werden, oder sie können andere zur Kristallisation verwendete Lösungsmittel einschliessen.

Die vorliegende Erfindung bezieht sich zusätzlich auf die Verwendung in Säugern der Verbindungen der Formel I und ihrer pharmazeutisch annehmbaren Salze, oder von pharmazeutischen Präparaten davon, als Inhibitoren serotonergischer Funktion, insbesondere als Serotonin-2-Blocker (Antagonisten von Serotonin an Serotonin-2-Rezeptoren), zur Behandlung von Krankheiten, welche auf Serotonin-2-Rezeptor-Blockade ansprechen, insbesondere von psychotropen Krankheiten, wie Angstzuständen, Depression oder Manien, von gastrointestinalen Krankheiten wie Geschwüren, und von cardiovaskulären Krankheiten wie Bluthochdruck.

Insbesondere bezieht sich die Erfindung auf ein Verfahren zur Inhibierung der Wirkung von Serotonin auf zentrale Serotonin-2-Rezeptoren und vorzugsweise auf ein Verfahren zur Behandlung von psychotropen Krankheiten in Säugern, z.B. solchen, die auf Serotonin-2-Blockade ansprechen, insbesondere Angstzuständen, unter Verwendung einer wirksamen Menge einer Verbindung der Erfindung, z.B. der Formel I, oder eines pharmazeutisch annehmbaren Salzes davon als pharmakologische Wirksubstanzen, vorzugsweise in der Form von pharmazeutischen Präparaten.

Die Dosis, in der die Wirksubstanzen verabreicht werden, ist abhängig von der Spezies des jeweiligen Warmblüters (Säugers), des Körpergewichts, des Alters und der individuellen Verfassung sowie von der Art der Verabreichung.

Eine Einheitsdosis für einen Säuger von 50 bis 70 kg dann zwischen etwa 1 und 50 mg der aktiven Wirksubstanz enthalten.

Die vorliegende Erfindung betrifft ebenfalls die Verwendung von Verbindungen der Erfindung zur Herstellung von pharmazeutischen Präparaten, insbesondere pharmazeutischen Präparaten mit modulierender Wirkung auf Serotonin-Rezeptoren, insbesondere mit Serotonin-2 blockierender Wirkung.

Die erfindungsgemässen pharmazeutischen Präparate eignen sich zur enteralen, wie oralen oder rektalen, transdermalen oder parenteralen Verabreichung an Säugetiere, inklusive Menschen, zur Behandlung von Krankheiten, die auf Antagonismus von Serotonin an Serotonin-Rezeptoren ansprechen. Diese Präparate enthalten eine wirksame Menge einer Verbindung der Formel I oder eines pharmazeutisch annehmbaren Salzes davon und zwar ohne Beimengung oder in Kombination mit einem oder mehreren pharmazeutisch annehmbaren Trägermaterialien.

Die pharmakologisch aktiven Verbindungen der Erfindung sind bei der Herstellung von pharmazeutischen Präparaten verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalz und/oder Polyethylenglycol, für Tabletten auch c) Bindemitteln, z.B. Magnesiumaluminiumsilicat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Adsorbentien, farbegebenden Mitteln, Geschmacksstoffen und süssenden Mitteln, umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvanzien, wie Konservierungs-, Stabiliserungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten vorzugsweise etwa 1 bis 50 % des aktiven Bestandteils.

Geeignete Formulierungen für die transdermale Anwendung enthalten eine wirksame Menge einer Verbindung der Formel I zusammen mit Trägermaterial. Geeignete Trägermaterialien umfassen pharmazeutisch annehmbare Hilfsstoffe, welche den Durchgang durch die Haut ermöglichen. Insbesondere haben transdermale therapeutische Systeme die Form eines Pflasters mit einer Schutzschicht, einem Wirkstoffreservoir, gegebenenfalls mit Trägermaterialien, und einer die Abgabe bestimmenden Kontrollschicht, durch welche der Wirkstoff auf die Haut mit kontrollierter und bestimmbarer Geschwindigkeit über einen längeren Zeitabschnitt abgegeben wird. Das System hat gegebenenfalls noch eine Klebschicht.

Die folgenden Beispiele sollen die Erfindung näher erläutern. Temperaturen werden in Grad Celsius angegeben. Wenn nicht anders angegeben, werden alle Verdampfungen unter vermindertem Druck ausgeführt, vorzugsweise zwischen etwa 2 und 13 kPa. Die Struktur von Endprodukten, Zwischenprodukten und Ausgangsverbindungen ist z.B. durch analytische Verfahren wie Mikroanalyse und spektroskopische Charakteristika gesichert (z.B. MS, IR, NMR).

Beispiel 1:

Ein Gemisch von 1,3,4,16b-Tetrahydro-2-methyl-1,4-dioxo-2H,10H-indolo[2,1-c]pyrazino[1,2-a][1,4]-benzodiazepin-16-carbonsäuremethylester (1,48 kg) und Tetrahydrofuran (52 L) wird in einer Stickstoffatmosphäre unter Rückfluss erhitzt, und dann wird eine Lösung von Diboran in Tetrahydrofuran (1M, 9,47 L) während eines Zeitraums von 1 Stunde zugegeben. Der Ansatz wird während 20 Stunden unter Rückfluss gehalten, danach wird er der Reihe nach mit Methanol (4 L)(langsame, vorsichtige Zugabe) und einer Lösung von Chlor-wasserstoff in Methanol (7,5 N, 1,6 L) versetzt. Das Gemisch wird gerührt, für 2 Stunden unter Rückfluss erhitzt und im Vakuum eingeengt, der erhaltene feste Rückstand wird mit Aceton (4 L) für eine Stunde verrieben, gesammelt, mit Aceton gewaschen (4 x 500 ml) und getrocknet (18 Stunden, 50°C/65 Pa). Man erhält so den 1,3,4,16b-Tetrahydro-2-methyl-2H,10H-indolo[2,1-c]pyrazino[1,2-a][1,4]-benzodiazepin-16-carbonsäuremethylester als Hydrochlorid, Fp. 272-273°C. Dieses Salz wird in Dichlormethan suspendiert, und aus reichend Ammoniumhydroxid (28 %) in Wasser wird zugegeben, so dass die freie Base freigesetzt wird. Das basische Gemisch wird während 45 Minuten gerührt, die wässrige Phase wird entfernt und abgetrennt und die organische Phase mit Wasser gewaschen. Das Dichlormethan wird im Vakuum abgezogen und der zurückbleibende Schaum in kochendem 2-Propanol aufgelöst. Die Lösung wird filtriert, um kleine Mengen unlöslichen Materials abzufiltrieren, dann wird sie über Nacht zur vollständigen Kristallisation abgekühlt. Der erhaltene Feststoff wird durch Umkristallisieren aus 2-Propanol gereinigt. Man lässt die Lösung abkühlen, während über Nacht gerührt wird. Die erhaltenen Kristalle werden durch Filtration gesammelt, mit 2-Propanol gewaschen und getrocknet (16 Stunden, 80°/400 Pa). Man erhält so den 1,3,4,16b-Tetrahydro-2-methyl-2H,10H-indolo[2,1-c]pyrazino[1,2-a][1,4]benzodiazepin-16-carbonsäuremethylester, Fp. 157-159°C, die Verbindung der Formel I, worin $R^1$ für Methyl steht, $R^2$ und $R^3$ Wasserstoff bedeuten und $R^4$ Methoxycarbonyl ist.

Das Hydrochloridsalz wird wie folgt hergestellt: Methanol (20 L) und 2,37 kg 1,3,4,16b-Tetrahydro-2-methyl-2H,10H-indolo[2,1-c]pyrazino[1,2-a][1,4]benzodiazepin-16-carbonsäuremethylester werden vereinigt, unter Rückfluss erhitzt, und die Mischung wird mit einer Lösung von Chlorwasserstoff in Methanol (7,6N, 870 ml) behandelt. Der Ansatz wird für 10 Minuten unter Rückfluss gehalten, man lässt ihn dann allmählich auf Raumtemperatur kommen und rührt über Nacht. Die Suspension wird filtriert und das feste Hydrochlorid wird mit Methanol (2 x 500 ml) gewaschen und getrocknet (18 Stunden, 80°C/13 Pa). Die erhaltene Verbindung wird in kochendes Wasser gegeben (31 L), und das Gemisch wird unter Rückflüss erhitzt, bis vollständigt Lösung eingetreten ist (20 Minuten). Das Heizen wird dann beendet, und das Gemisch wird über Nacht bei 15°C gerührt, auf 5° abgekühlt, filtriert, und die gesammelten Feststoffe werden mit Wasser gewaschen (1 L). Das Produkt wird getrocknet (6 Stunden, 80°C/400 Pa; 30 Stunden, 100°C/13 Pa), durch ein Sieb von 40 mesh getrieben und erneut getrocknet (15 Stunden, 110°C/13 Pa). Man erhält so den 1,3,4,16b-Tetrahydro-2-methyl-2H,10H-indolo[2,1-c]pyrazino[1,2-a][1,4]benzodiazepin-16-carbonsäuremethylester als Hydrochlorid, Fp. 275-277°C.

Das Ausgangsmaterial wird wie folgt hergestellt: Eine Lösung von Trichloracetylchlorid (5,60 kg) in Dichlormethan (3,5 L) wird über einen Zeitraum von 5,5 Stunden zu einer kalten (5-10°C) gerührten Mischung von Indol (3,50 kg) und Pyridin (2,53 kg) in Dichlormethan (15 L) gegeben. Das Reaktionsgemisch wird 48 Stunden gerührt und danach filtriert, um den rohen Feststoff zu sammeln. Das Filtrat wird auf 5 L eingeengt, und eine zweite Menge roher Feststoff wird abfiltriert. Beide Mengen werden vereinigt und mit 1:1 Ethanol/Wasser (18 L) verrieben. Nach dem Trocknen (18 Stunden, 80°C/133 Pa) wird 3-Trichloracetyl-1H-indol erhalten, Fp. 231-234°C (Zersetzung).

Natriumstücke (0,106 kg) werden vorsichtig portionsweise zu gerührtem Methanol (20,0 L) unter einer Stickstoffatmosphäre gegeben. Wenn die Umsetzung vollständig ist, wird die Lösung auf 25°C gekühlt und 3-Trichloracetyl-1H-indol (5,94 kg) wird portionsweise über einen Zeitraum von 35 Minuten zugegeben. Das Gemisch wird für 2 Stunden gerührt, auf 10° abgekühlt, worauf eiskaltes Wasser (20 L) zugegeben wird. Kristallines Produkt wird durch Filtrieren isoliert, mit 1:1 Methanol/Wasser (2 L) gewaschen und getrocknet (12 Stunden, 60°C/400 Pa; 36 Stunden, 25°C/13 Pa). Man erhält so den 1H-Indol-3-carbonsäuremethylester, Fp. 146-148°C.

Lithiumamid (0,416 kg) wird portionsweise zu einer gerührten Lösung von 1H-Indol-3-carbonsäuremethylester (3,17 kg) in Dimethylsulfoxid (27 L) bei anfänglich 20°C unter einer Stickstoffatmosphäre unter äusserem Kühlen gegeben. Diese Mischung wird eine Stunde gerührt, dann wird 2-Nitrobenzylchlorid (3,10 kg) in 2 Portionen innerhalb einer Stunde zugegeben. Nach vollständiger Zugabe wird die Mischun g für 2 Stunden bei Raumtemperatur gerührt und dann in heftig gerührtes eiskaltes Wasser (80 L) gegeben. Der Niederschlag wird durch Filtration gesammelt, mit zum Rückfluss erhitztem 2-Propanol (12 L) während einer Stunde verrieben, filtriert und über Nacht luftgetrocknet, wobei 1-(2-Nitrobenzyl)-1H-indol-3-carbonsäuremethylester erhalten wird, Fp. 129-132°C.

1-(2-Nitrobenzyl)-1H-indol-3-carbonsäuremethylester (4,50 kg), Eisessig (27 L) und Wasser (2,7 L) werden vereinigt und auf 65° C erhitzt. Eisenfeilspäne (3,60 kg) werden in 8 Portionen während eines Zeitraums von 3 Stunden zugegeben. Nach jeder Zugabe wird das Gemisch auf etwa 80° C gekühlt. Nach beendeter Zugabe wird das Gemisch während 2 Stunden bei 85-90° C gehalten, dann mit Wasser (40 L) verdünnt, filtriert, und der gesammelte Feststoff wird mit Wasser gewaschen (6 x 4 L), über Nacht luftgetrocknet, in Dimethylformamid (32 L) gelöst, und die Lösung wird filtriert um unlösliches Material zu entfernen. Das Filtrat wird auf Wasser (72 L) gegeben und das Gemisch wird 30 Minuten gerührt, filtriert, mit Wasser (10 x 3 L) gewaschen und getrocknet (120 Stunden, 60° C/65 Pa), worauf der 1-(2-Aminobenzyl)-1H-indol-3-carbonsäuremethylester erhalten wird, Fp. 168-170° C.

Ethyloxalylchlorid (2,68 kg) wird während 2,5 Stunden zu einer gerührten Mischung von 1-(2-Aminobenzyl)-1H-indol-3-carbonsäuremethylester (5,00 kg), Pyridin (1,55 kg) und Dichlormethan (50 L) bei Raumtemperatur unter einer Stickstoffatmosphäre gegeben. Der Ansatz wird dann über Nacht gerührt, mit 1N Salzsäure (10 L) gewaschen und mit Wasser (2 x 15 L), wobei jede Waschflüssigkeit von der unteren organischen Phase abgetrennt wird. Die Aufschlämmung des gewünschten Produkts in Dichlormethan wird unter Vakuum eingeengt, man erhält so einen Feststoff, der mit 2-Propanol (6 L) verrieben wird, er wird gesammelt und getrocknet (8 Stunden, 70° C/400 Pa; 48 Stunden, 70° C/13 Pa). Man erhält so den 1-[2-(Ethoxycarbonylcarbonylamino)benzyl]-1H-indol-3-carbonsäuremethylester; Fp. 174-177° C.

Phosphorpentoxid (1,00 kg) wird zu einer gerührten Suspension von 1-[2-(Ethoxycarbonylcarbonylamino)benzyl]-1H-indol-3-carbonsäuremethylester (1,00 kg) in Phosphoroxychlorid (6 L) bei Raumtemperatur gegeben. Der Ansatz wird für 30 Minuten gerührt und dann für eine Stunde auf 100° C erhitzt, wonach der Hauptteil des Lösungsmittels durch Destillation bei vermindertem Druck entfernt wird. Der ölige Rückstand wird in Dichlormethan (6 L) aufgenommen, und die Lösung wird vorsichtig in eine gerührte Mischung von Eis (20 kg) mit Wasser (20 L) gegeben. Zusätzliches Dichlormethan (16 L) wird zugesetzt, und das Gemisch wird eine Stunde gerührt. Die organische Phase wird abgetrennt, und die wässrige Phase wird mit Dichlormethan (3 x 4 L) extrahiert. Die vereinigten Extrakte werden im Vakuum eingeengt, man erhält so einen Feststoff, der bei 95° C in Toluol aufgenommen wird, das Gemisch wird 3 Stunden gerührt und heiss filtriert. Das Filtrat wird im Vakuum eingeengt, der feste Rückstand wird mit Diethylether verrieben, gesammelt und getrocknet (16 Stunden, 25° C/13 Pa). Man erhält so den 6H-Indolo-[2,1-c][1,4]benzodiazepin-12,13-dicarbonsäure-12-ethyl-13-methylester, Fp. 188-190° C.

Eine Lösung von Natriumhypophosphitmonohydrat (3,34 kg) in Wasser (16 L) wird während eines Zeitraums von 7 Stunden zu einer gerührten heissen (50-55° C) Mischung von 6H-Indolo[2,1-c][1,4]-benzodiazepin-12,13-dicarbonsäure-12-ethyl-13-methylester (3,45 kg), 5 % Palladium auf Kohle (50 % feucht, 286 g) und Kaliumcarbonat (3,34 kg) in Tetrahydrofuran (23 L) unter einer Stickstoffatmosphäre gegeben. Nach vollständiger Zugabe lässt man den Ansatz allmählich abkühlen, wobei über Nacht gerührt wird. Die Vollständigkeit der Umsetzung wird durch Dünnschichtchromatographie auf Silikagelplatten verfolgt (85:15 Toluol/Essigester), bevor aufgearbeitet wird. Wenn die Reduktion unvollständig ist, wird mehr Carbonat und Hypophosphit zugegeben, und man lässt den Ansatz für einen weiteren Zeitraum reagieren. Die Ph asen werden getrennt, und die wässrige Phase wird mit Essigester (2 x 8 L) extrahiert. Die vereinigten organischen Phasen werden filtriert und im Vakuum eingeengt. Man erhält so einen Feststoff, der mit Diethylether (4 L) verrieben wird, filtriert und trocknet (6 Stunden, 60° C/400 Pa; 18 Stunden, 60° C/13 Pa). Man erhält so den 11,12-Dihydro-6H-indolo[2,1-c][1,4]benzodiazepin-12,13-dicarbonsäure-12ethyl-13-methylester, Fp. 184-186° C.

Ein Gemisch von 11,12-Dihydro-6H-indolo[2,1-c][1,4]benzodiazepin-12,13-dicarbonsäure-12ethyl-13-methylester (3,40 kg) und Chloracetylchlorid (1,16 kg) in Essigester (28 L) wird 5 Stunden unter Rückfluss erhitzt. Man lässt den Ansatz dann abkühlen, wobei über Nacht gerührt wird. Der Ansatz wird filtriert, man erhält so einen Feststoff, der mit Diethylether (2 x 1 L) gewaschen wird und dann getrocknet (18 Stunden, 80° C/400 Pa). Man erhält so den 11-Chloracetyl-11,12-dihydro-6H-indolo[2,1-c][1,4]benzodiazepin-12,13-dicarbonsäure-12-ethyl-13-methylester, Fp. 231-233° C. Eine weitere Menge dieser Verbindung wird durch Konzentrieren des Filtrats auf ein geringes Volumen erhalten.

Eine gerührte Mischung von 11-(Chloracetyl)-11,12-dihydro-6H-indolo[2,1-c][1,4]benzodiazepin-12,13-dicarbonsäure-12-ethyl-13-methylester (6,93 kg) in Tetrahydrofuran (46 L) wird auf 5° C gekühlt. Dann wird gasförmiges Monomethylamin (5,00 kg) in einem stetigem Strom unter die Oberfläche der Lösung eingeleitet. Das Gemisch wird über Nacht bei Raumtemperatur gerührt, 3 Stunden unter Rückfluss erhitzt, auf 20° abgekühlt und filtriert. Der erhaltene Feststof wird dann auf dem Filter mit Tetrahydrofuran (4 x 1 L) gewaschen und mit Wasser (20 L) während einer Stunde verrieben. Die Suspension wird filtriert und mit Wasser (6 x 4 L) gewaschen. Der Feststoff wird getrocknet (18 Stunden, 100° C/6 Pa). Man erhält so 1,3,4,16b-Tetrahydro-2-methyl-1,4-dioxo-2H,10H-indolo[2,1-c][pyrazino[1,2-a][1,4]benzodiazepin-16-carbonsäuremethylester, Fp. 357° C (Zersetzung).

Beispiel 2:

Zu einer Lösung von 1 g 1,3,4,16b-Tetrahydro-2-methyl-2H,10H-indolo[2,1-c]pyrazino[1,2-a][1,4]-benzodiazepin-16-carbonsäuremethylester in 25 ml Ethanol werden 15 ml einer 5N Kaliumhydroxidlösung gegeben, und der Ansatz wird 3 Stunden unter Rückfluss erhitzt. Nahc dem Abkühlen wird unter vermindertem Druck das Ethanol abgezogen. Der wässrige Rückstand wird mit 15 ml Wasser verdünnt, und die Lösung wird mit Essigsäure auf den pH 6 eingestellt. Der Niederschlag von 1,3,4,16b-Tetrahydro-2-methyl-2H,10H-indolo[2,1-c]pyrazino[1,2-a][1,4]benzodiazepin-16-carbonsäure wird gesammelt. Nach Kristallisieren aus Aceton weist er einen Fp. von 280-284° C auf.

Beispiel 3:

Reduktion von 1,3,4,16b-Tetrahydro-1,4-dioxo-2-methyl-2H,10H-indolo[2,1-c][pyrazino[1,2-a][1,4]-benzodiazepin mit Diboran auf analoge Weise wie in Beipiel 1 beschrieben, ergibt 1,3,4,16b-Tetrahydro-2-methyl-2H,10H-indolo[2,1-c][pyrazino[1,2-a][1,4]benzodiazepin als Hydrochlorid, Fp. 279-280° C.

Das Ausgangsmaterial wird wie folgt hergestellt: Ein Gemisch von 9,5 g 1,3,4,16b-Tetrahydro-2-methyl-1,4-dioxo-2H,10H-indolo[2,1-c][pyrazino[1,2-a][1,4]benzodiazepin-16-carbonsäuremethylester, 95 ml Ethanol und 190 ml 1N Natriumhydroxid wird unter Rühren für 2 Stunden unter Rückfluss erhitzt. Das Lösungsmittel wird dann unter vermindertem Druck abgezogen, man erhält so das Natriumsalz der entsprechenden Carbonsäure. Zu diesem Rückstand werden 95 ml 6N Salzsäure und 190 ml Tetrahydrofuran gegeben, und der Ansatz wird für 5 Stunden unter Rückfluss erhitzt. Nach Kühlen auf Raumtemperatur wird unter vermindertem Druck das Lösungsmittel bis zur Trockne abgezogen, dann wird mit einem Gemisch von 200 ml Dichlormethan und 100 ml 1N Natriumhydroxid extrahiert. Der Dichlormethanextrakt wird abgetrennt und mit 50 ml gesättigter Natriumchloridlösung gewaschen. Die Dichlormethanlösung wird dann über wasserfreiem Magnesiumsulfat getrocknet, filtriert, und das Lösungsmittel unter vermindertem Druck abgezogen. Man erhält so 1,3,4,16b-Tetrahydro-1,4-dioxo-2-methyl-2H,10H-indolo[2,1-c]pyrazino[1,2-a][1,4]-benzodiazepin, das aus Ether kristallisiert wird, Fp. 188-189° C.

Beispiel 4:

Zu dem Reagenz aus 0,56 ml Dimethylformamid in 5 ml Dichlormethan und 1,03 g Phosphoroxychlorid wird eine Lösung von 1,88 g 1,3,4,16b-Tetrahydro-2-methyl-2H,10H-indolo[2,1-c][pyrazino[1,2-a][1,4]-benzodiazepin in 5 ml Dichlormethan tropfenweise gegeben, wobei die Temperatur des Ansatzes unterhalb von 10° C gehalten wird. Man lässt den Ansatz 30 Minuten bei Raumtemperatur rühren. Der Ansatz wird mit 10 ml einer gesättigten Lösung von Natriumacetat versetzt und eine Stunde unter Rückfluss erhitzt. Nach Abkühlen auf Raumtemperatur wird der Ansatz mit Dichlormethan (3 x 50 ml) extrahiert. Die organischen Extrakte werden vereinigt und über wasserfreiem Natriumsulfat getrocknet, das Lösungsmittel wird unter vermindertem Druck durch Eindampfen entfernt. Man erhält so 1,3,4,16b-Tetrahydro-2-methyl-2H,10H-indolo[2,1-c][pyrazino[1,2-a][1,4]benzodiazepin-16-carboxaldehyd, der in das Hydrochloridsalz übergeführt wird, Fp. 260-266° C.

Beispiel 5:

Zu einer Lösung von 5,4 g 1,3,4,16-Tetrahydro-2-methyl-2H,10H-indolo[2,1-c]pyrazino[1,2-a][1,4]-benzodiazepin-16-carbonsäuremethylester in 100 ml Tetrahydrofuran wird 1 g Lithiumaluminiumhydrid während 10 Minuten in kleinen Portionen gegeben, wobei die Temperatur des Ansatzes unter 25° C gehalten wird. Nach vollständiger Zugabe wird der Ansatz 4 Stunden unter Rückfluss erhitzt. Nach Abkühlen auf Raumtemperatur wird der Ansatz vorsichtig tropfenweise mit 0,65 ml Wasser versetzt, dann mit 0,65 ml 15 % Natriumhydroxidlösung, gefolgt von 1,95 ml Wasser. Der Ansatz wird filtriert, und der Filterkuchen heftig mit 100 ml Dichlormethan gewaschen. Das Filtrat wird unter vermindertem Druck zur Trockne eingeengt, man erhält so 1,3,4-16b-Tetrahydro-2-methyl-16-hydroxymethyl-2H,10H-indolo[2,1-c]-pyrazino[1,2-a][1,4]benzodiazepin als Oel, welches, nach Kristallisieren aus Ether, einen Fp. von 184-189° C aufweist.

Beispiel 6:

Zu einer Lösung von 4,4 g 1,3,4,16b-Tetrahydro-2-ethoxycarbonyl-2H,10H-indolo[2,1-c]pyrazino[1,2-a]-[1,4]benzodiazepin in 88 ml Ethanol werden 24 ml einer 50 %igen Kaliumhydroxidlösung gegeben. Der

Ansatz wird 6 Stunden unter Rückfluss erhitzt. Das Lösungsmittel wird unter vermindertem Druck zur Trockne abgezogen, der Rückstand wird mit einem Gemisch von 350 ml Diethylether und 250 ml Wasser extrahiert. Der etherische Extrakt wird mit Wasser gewaschen, abgetrennt und dann über wasserfreiem Magnesiumsulfat getrocknet. Das Lösungsmittel wird abgezogen, man erhält so 1,3,4,16b-Tetrahydro-2H,10H-indolo[2,1-c]pyrazino[1,2-a][1,4]benzodiazepin, welches in das Hydrochloridsalz übergeführt wird, Fp. 263 °C (Zersetzung).

Das Ausgangsmaterial wird wie folgt hergestellt: Zu einer Lösung von 712 mg 1,3,4,16-Tetrahydro-2-methyl-2H,10H-indolo[2,1-c]pyrazino[1,2-a][1,4]benzodiazepin in 5 ml Toluol werden 1,2 g Chlorameisensäureethylester gegeben, und das Gemisch wird 8 Stunden unter Rühren unter Rückfluss erhitzt. Nach Abkühlen auf Raumtemperatur wird der Ansatz mit 50 ml Diethylether verdünnt, und der erhaltene Niederschlag wird durch Filtrieren gesammelt und gut mit Ether gewaschen. Das Filtrat wird unter vermindertem Druck zur Trockne eingeengt. Man erhält so 1,3,4,16b-Tetrahydro-2-ethoxycarbonyl-2H,10H-indolo[2,1-c]pyrazino[1,2-a][1,4]benzodiazepin als Oel, das in der nächsten Stufe ohne weitere Reinigung eingesetzt wird.

Beispiel 7:

Zu einer Lösung von 1,4 g 1,3,4,16b-Tetrahydro-2-methyl-2H,10H-indolo[2,1-c]pyrazino[1,2-a][1,4]-benzodiazepin-16-carboxaldehyd in 35 ml Tetrahydrofuran werden 17 ml 1M Diboran in Tetrahydrofuran gegeben. Der Ansatz wird 5 Stunden unter Rückfluss erhitzt. Der Ansatz wird dann mit 10 ml Methanol und 3,5 ml Essigsäure versetzt, und das Heizen wird für eine weitere Stunde fortgeführt. Das Lösungsmittel wird unter vermindertem Druck abgezogen, der Rückstand wird in Dichlormethan aufgenommen und mit 5%iger Natriumhydroxidlösung gewaschen. Die Dichlormethanphase wird abgetrennt, das Lösungsmittel unter vermindertem Druck abgezogen, man erhält so 1,3,4,16b-Tetrahydro-2,16-dimethyl-2H,10H-indolo[2,1-c]-pyrazino[1,2-a][1,4]benzodiazepin, welches in das Hydrochloridsalz übergeführt wird, Fp. 293-298 °C (Zersetzung).

Beispiel 8:

Die folgenden Verbindungen der Formel I werden auf analoge Weise zu denen, die in den vorstehenden Beispielen beschrieben sind, hergestellt:

a) 1,3,4,16b-Tetrahydro-2H,10H-indolo[2,1-c]pyrazino[1,2-a][1,4]benzodiazepin-16-carbonsäuremethylesterhydrochlorid, Fp. 236-240 °C;

b) 1,3,4,16b-Tetrahydro-2-n-propyl-2H,10H-indolo[2,1-c]pyrazino[1,2-a][1,4]benzodiazepinhydrochlorid, Fp. 258-260 °C (Zersetzung);

c) 1,3,4,16b-Tetrahydro-16-hydroxymethyl-2-n-propyl-2H,10H-indolo[2,1-c]pyrazino[1,2-a][1,4]-benzodiazepinhydrochlorid, Fp. 186-190 °C;

d) 1,3,4,16b-Tetrahydro-2H,10H-indolo[2,1-c]pyrazino[1,2-a][1,4]benzodiazepin-16-carboxamid;

e) 1,3,4,16b-Tetrahydro-2-dimethylaminoethyl-2H,10H-indolo[2,1-c]pyrazino[1,2-a][1,4]benzodiazepin-16-carbonsäuremethylester;

f) 1,3,4,16b-Tetrahydro-2-n-propyl-2H,10H-indolo[2,1-c]pyrazino[1,2-a][1,4]benzodiazepin-16-carbonsäuremethylesterhydrochlorid, Fp. 258-260 °C;

g) 1,3,4,16b-Tetrahydro-14-methoxy-2-methyl-2H,10H-indolo[2,1-c]pyrazino[1,2-a][1,4]-benzodiazepinhydrochlorid, Fp. 280 °C (Zersetzung);

h) 1,3,4,16b-Tetrahydro-14-chlor-2-methyl-2H,10H-indolo[2,1-c]pyrazino[1,2-a][1,4]benzodiazepin-16-carbonsäuremethylesterhydrochlorid, Fp. 266 °C (Zersetzung);

i) 1,3,4,16b-Tetrahydro-14-methoxy-2-methyl-2H,10H-indolo[2,1-c]pyrazino[1,2-a][1,4]benzodiazepin-16-carbonsäuremethylesterhydrochlorid, Fp. 271 °C (Zersetzung);

j) 1,3,4,16b-Tetrahydro-7-chlor-2-methyl-2H,10H-indolo[2,1-c]pyrazino[1,2-a][1,4]benzodiazepin-16-carbonsäuremethylesterhydrochlorid, Fp. 237 °C (Zersetzung);

k) 1,3,4,16b-Tetrahydro-14-chlor-2-methyl-2H,10H-indolo[2,1-c]pyrazino[1,2-a][1,4]benzodiazepin.

Die folgenden Ausgangsmaterialien werden z.B. nach den Verfahren die in den vorstehenden Beispielen beschrieben sind, hergestellt:

1) 1,3,4,16b-Tetrahydro-1,4-dioxo-2H,10H-indolo[2,1-c]pyrazino[1,2-a][1,4]benzodiazepin-16-carbonsäuremethylester, Fp. 291 °C (das Material für Verbindung a);

2) 1,3,4,16b-Tetrahydro-1,4-dioxo-2-n-propyl-2H,10H-indolo[2,1-c]pyrazino[1,2-a][1,4]benzodiazepin-16-carbonsäuremethylester, Fp. 298-300 °C (das Ausgangsmaterial für Verbindungen b und c);

3) 1,3,4,16b-Tetrahydro-1,4-dioxo-2H,10H-indolo[2,1-c]pyrazino[1,2-a][1,4]benzodiazepin-16-carboxamid,

Fp. 292° C (das Ausgangsmaterial für Verbindung d), welches durch Behandeln des Ausgangsmaterials 1 wie vorstehend mit übschüssigem wasserfreiem Ammoniak erhalten werden kann, oder als Nebenprodukt bei der Herstellung des Ausgangsmaterials 1 gemäss dem allgemeinen Verfahren, das für das Ausgangsmaterial in Beispiel 1 beschrieben ist, wobei aber im letzten Schritt wasserfreies Ammoniak verwendet wird:

4) 1,3,4,16b-Tetrahydro-1,4-dioxo-2-dimethylaminoethyl-2H,10H-indolo[2,1-c]pyrazino[1,2-a][1,4]-benzodiazepin-16-carbonsäuremethylester, Fp. 222-224° C (das Ausgangsmaterial für Verbindung e);

5) Das 1,4-Dioxo Ausgangsmaterial für die Verbindungen g, h, i and k aus den entsprechend 5-substituierten Indolen gemäss dem Verfahren, welches in Beispiel 1 beschrieben ist;

6) Das 1,4-Dioxo substituierte Ausgangsmaterial für Verbindung j gemäss dem Verfahren, welches in Beispiel 1 beschrieben ist, wobei man von 4-Chlor-2-nitrobenzylchlorid ausgeht.

Beispiel 9:

a) Herstellung von 10.000 Tabletten mit je 10,0 mg Wirkstoff:

Bestandteile

1,3,4,16b-Tetrahydro-2-methyl-2H,10H-indolo[2,1-c]pyrazino[1,2-a][14]benzodiazepin-16-carbonsäuremethylester Hydrochlorid 100,00 g
Lactose 2.535,00 g
Maisstärke 125,00 g
Polyethylenglycol 6,000 150,00 g
Magnesiumstearat 40,00 g
Wasser (steril) q.s.

Das pulverige Material wird durch ein Sieb mit Oeffnungen von 0,6 mm gedrückt. Anschliessend mischt man in einem Mischer den Wirkstoff mit der Lactose, dem Magnesiumstearat und der halben Menge Stärke. Die andere halbe Menge Stärke wird in 65 ml Wasser suspendiert, und diese Suspension wird zu der siedenden Lösung des Polyethylenglycols in 260 ml in Wasser gegeben. Die gebildete Paste wird zu der Pulvermischung gegeben, welche gegebenenfalls mit weiterem Wasser granuliert wird. Man trocknet das Granulat über Nacht bei 35° C, treibt dieses durch ein Sieb mit 1,2 mm weiten Oeffnungen und komprimiert zu konkaven Tabletten mit oberer Bruchkerbe.

Auf analoge Weise werden Tabletten hergestellt, die etwa 1-50 mg einer der anderen vorstehend offenbarten Verbindungen enthalten.

b) Herstellung von 1.000 Kapseln mit je 5 mg Wirkstoff:

Bestandteile

1,3,4,16b-Tetrahydro-2-methyl-2H,10H-indolo[2,1-c]pyrazino[1,2-a][1,4]benzodiazepin-16-carbonsäuremethylester Hydrochlorid 5,00 g
Lactose 212,00 g
Stärke 80,00 g
Magnesiumstearat 3,00 g

Das pulverige Material wird durch ein Sieb mit Oeffnungen von 0,6 mm Durchmesser gedrückt. Anschliessend mischt man in einem Mischer den Wirkstoff mit dem Magnesiumstearat, dann mit der Lactose und der Stärke, bis man eine homogene Masse erhält. Man füllt Nr. 2 Hartgelatinekapseln mit jeweils 300 mg der vorbereiteten Mischung.

Analog kann man Kapseln, welche 1 - 50 mg einer der anderen vorstehend offenbarten Verbindungen enthalten, herstellen.

**Patentansprüche**

**Patentansprüche für folgende Vertragsstaaten : BE, CH, DE, FR, GB, IT, LI, LU, NL, SE**

**1.** Verbindungen der Formel I,

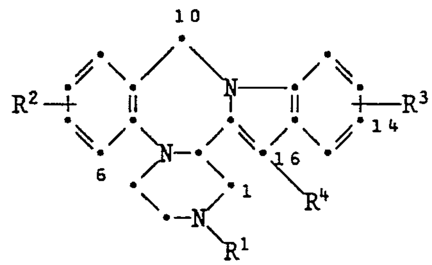

(I)

worin $R^1$ Wasserstoff, Niederalkyl, $C_3$-$C_7$-Alkenyl, welches über ein gesättigtes Kohlenstoffatom gebunden ist, $C_3$-$C_7$-Alkinyl, welches über ein gesättigtes Kohlenstoffatom gebunden ist, 3 bis 7-gliedriges Cycloalkyl, oder $C_2$-$C_7$-Alkyl, welches durch einen Substituenten ausgewählt aus der Gruppe bestehend aus Hydroxy, Amino, N-Mononiederalkylamino und N,N-Diniederalkylamino substituiert ist, wobei diese Substituenten vom Ringstickstoffatom durch mindestens 2 Kohlenstoffatome getrennt sind, bedeutet; oder worin $R^1$ Niederalkyl, welches durch einen Substituenten ausgewählt aus der Gruppe bestehend aus 3 bis 7-gliedrigem Cycloalkyl, Carboxy, Niederalkoxycarbonyl, Carbamoyl, N-Mononiederalkylcarbamoyl, N,N-Diniederalkylcarbamoyl und Niederalkanoyl substituiert ist, bedeutet; oder worin $R^1$ Niederalkyl, welches durch Phenyl oder Benzoyl substituiert ist, bedeutet, wobei jedes dieser Phenyl- oder Benzoylradikale unsubstituiert oder durch bis zu drei Substituenten ausgewählt aus der Gruppe bestehend aus Niederalkyl, Niederalkoxy, Niederalkylthio, Halogen und Trifluormethyl substituiert ist; worin $R^2$ und $R^3$, unabhängig voneinander, für Wasserstoff, Niederalkyl, Hydroxy, Niederalkoxy, Halogen oder Trifluormethyl stehen und $R^4$ für Wasserstoff, Niederalkyl, Hydroxyniederalkyl, Carboxy, Niederalkoxycarbonyl, Carbamoyl, N-Mono- oder N,N-Diniederalkylcarbamoyl oder Formyl steht; wobei der Ausdruck "Nieder" Radikale mit bis zu und einschliesslich 7 Kohlenstoffatomen bezeichnet; Salze, insbesondere pharmazeutisch annehmbare Salze davon; und die 2-N-Oxide dieser Verbindungen, worin $R^1$ die vorstehend angegebenen Bedeutungen aufweist aber nicht Wasserstoff bedeutet.

2. Verbindungen der Formel I gemäss Anspruch 1, worin $R^1$ Wasserstoff, Niederalkyl, $C_3$-$C_7$-Alkenyl, welches über ein gesättigtes Kohlenstoffatom gebunden ist, oder $C_2$-$C_7$-Alkyl, welches durch Hydroxy substituiert ist, bedeutet; worin $R^2$ und $R^3$, unabhängig voneinander, für Wasserstoff, Niederalkyl, Niederalkoxy, Halogen oder Trifluormethyl stehen und $R^4$ für Wasserstoff, Niederalkyl, Hydroxymethyl, Carboxy, Niederalkoxycarbonyl, Carbamoyl, oder N-Mono- oder N,N-Diniederalkylcarbamoyl steht; pharmazeutisch annehmbare Salze davon; und die 2-N-Oxide dieser Verbindungen, worin $R^1$ die vorstehend angegebenen Bedeutungen aufweist aber nicht Wasserstoff bedeutet.

3. Verbindungen der Formel I gemäss Anspruch 1, worin $R^1$ Wasserstoff oder Niederalkyl bedeutet; $R^2$ für Wasserstoff, Niederalkyl, Halogen oder Trifluormethyl steht; $R^3$ für Wasserstoff, Niederalkyl, Niederalkoxy, Halogen oder Trifluormethyl steht; $R^4$ für Wasserstoff, Niederalkyl, Hydroxymethyl, Carboxy, Niederalkoxycarbonyl, Carbamoyl oder N-Mono-oder N,N-Diniederalkylcarbamoyl steht; und pharmazeutisch annehmbare Salze davon.

4. Verbindungen der Formel I gemäss Anspruch 1, worin $R^1$ Wasserstoff oder Niederalkyl bedeutet; $R^2$ für Wasserstoff oder Halogen steht; $R^3$ für Wasserstoff, Niederalkoxy oder Halogen steht; $R^4$ für Carboxy, Niederalkoxycarbonyl, Carbamoyl oder N-Mono- oder N,N-Diniederalkylcarbamoyl steht; und pharmazeutisch annehmbare Salze davon.

5. Verbindungen der Formel I gemäss Anspruch 1, worin $R^1$ Wasserstoff oder Niederalkyl bedeutet; $R^2$ für Wasserstoff oder Halogen steht; $R^3$ für Wasserstoff, Niederalkoxy oder Halogen steht; $R^4$ für Niederalkoxycarbonyl oder Carbamoyl steht; und pharmazeutisch annehmbare Salze davon.

6. Verbindungen der Formel I gemäss Anspruch 1, worin $R^1$ Niederalkyl bedeutet; $R^2$ und $R^3$ für Wasserstoff stehen; $R^4$ für Niederalkoxycarbonyl steht; und pharmazeutisch annehmbare Salze davon.

7. 1,3,4,16b-Tetrahydro-2-methyl-2H,10H-indolo[2,1-c]pyrazino[1,2-a]-[1,4]benzodiazepin oder ein pharmazeutisch annehmbares Salz davon.

**8.** 1,3,4,16b-Tetrahydro-2-methyl-2H,10H-indolo[2,1-c]pyrazino[1,2-a]-[1,4]benzodiazepin-16-carbonsäuremethylester oder ein pharmazeutisch annehmbares Salz davon.

**9.** Verbindungen der Formel IIA

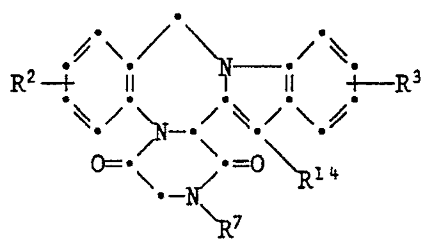

(IIA)

worin $R^7$ Wasserstoff, Niederalkyl, $C_3$-$C_7$-Alkenyl, welches über ein gesättigtes Kohlenstoffatom gebunden ist, $C_3$-$C_7$-Alkinyl, welches über ein gesättigtes Kohlenstoffatom gebunden ist, 3 bis 7-gliedriges Cycloalkyl, $C_2$-$C_7$-Alkyl, welches durch einen Substituenten ausgewählt aus der Gruppe bestehend aus Hydroxy, Amino, N-Mononiederalkylamino und N,N-Diniederalkylamino substituiert ist, wobei diese Substituenten vom Ringstickstoffatom durch mindestens 2 Kohlenstoffatome getrennt sind, bedeutet; oder worin $R^7$ Niederalkyl, welches durch einen Substituenten substituiert ist, welcher ausgewählt ist aus der Gruppe bestehend aus 3 bis 7-gliedrigem Cycloalkyl, Niederalkoxycarbonyl, Carbamoyl, und Phenyl, welches unsubstituiert oder durch bis zu 3 Substituenten ausgewählt aus der Gruppe Niederalkyl, Niederalkoxy, Niederalkylthio, Halogen und Trifluormethyl substituiert ist, bedeutet; $R^2$ und $R^3$ unabhängig voneinander Wasserstoff, Niederalkyl, Hydroxy, Niederalkoxy, Halogen oder Trifluormethyl bedeuten; und $R^{14}$ für Wasserstoff, Niederalkyl, Hydroxyniederalkyl, Niederalkoxycarbonyl oder Carbamoyl steht; wobei der Ausdruck "Nieder" Radikale mit bis zu ein schließlich 7 Kohlenstoffatomen bezeichnet; und Salze davon.

**10.** Verbindungen der Formel IIA gemäss Anspruch 9, worin $R^7$ für Wasserstoff oder Niederalkyl steht, $R^2$ Wasserstoff, Niederalkyl, Halogen oder Trifluormethyl bedeutet, $R^3$ für Wasserstoff, Niederalkyl, Niederalkoxy, Halogen oder Trifluormethyl steht, und $R^{14}$ Wasserstoff, Niederalkyl oder Niederalkoxycarbonyl bedeutet, und Salze davon.

**11.** Pharmazeutische Präparate enthaltend eine oder mehrere der in den Ansprüchen 1 bis 8 genannten Verbindungen.

**12.** Pharmazeutische Präparate enthaltend eine oder mehrere der in den Ansprüchen 1 bis 8 genannten Verbindungen zusammen mit einem oder mehreren pharmazeutisch geeigneten Trägermaterialien.

**13.** Die in den Ansprüchen 1 bis 8 genannten Verbindungen zur Anwendung in einem Verfahren zur therapeutischen Behandlung des menschlichen oder tierischen Körpers.

**14.** Die in den Ansprüchen 1 bis 8 genannten Verbindungen als Serotonin-2-Rezeptor-Antagonisten.

**15.** Verwendung der in den Ansprüchen 1 bis 8 genannten Verbindungen zur Herstellung von pharmazeutischen Präparaten.

**16.** Verwendung der in den Ansprüchen 1 bis 8 genannten Verbindungen zur Herstellung von pharmazeutischen Präparaten zur Verwendung als Serotonin-2-Rezeptor-Antagonisten.

**17.** Verfahren zur Herstellung eines pharmazeutischen Präparates, gekennzeichnet durch die Verarbeitung eines erfindungsgemässen Wirkstoffs nach einem der Ansprüche 1 bis 8 mit einem pharmazeutischen Trägermaterial.

**18.** Verfahren zur Herstellung von Verbindungen der Formel I gemäss Anspruch 1, gekennzeichnet durch die folgenden Verfahrensschritte:
a) eine Verbindung der Formel II,

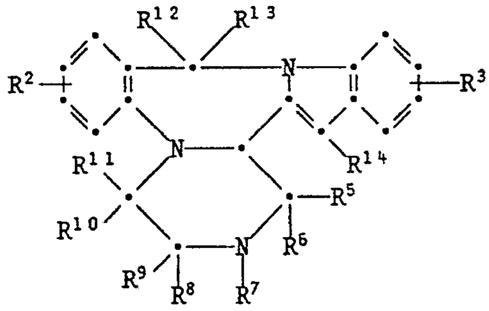

(II)

worin $R^2$ und $R^3$ die vorstehend angegebenen Bedeutungen aufweisen; $R^5$ und $R^6$ Wasserstoff bedeuten oder $R^5$ und $R^6$ zusammen Oxo bedeuten; $R^7$ die Bedeutung von $R^1$ hat und vorzugsweise für Wasserstoff, Niederalkyl, $C_2$-$C_7$-Alkyl, welches durch Hydroxy, Amino oder Mono- oder Diniederalkylamino substituiert ist, $C_3$-$C_7$-Alkenyl, $C_3$-$C_7$-Alkinyl, oder Niederalkyl, welches durch 3 bis 7-gliedriges Cycloalkyl oder durch gegebenenfalls substituiertes Phenyl substituiert ist, steht; $R^8$ und $R^9$ Wasserstoff bedeuten oder $R^8$ und $R^9$ zusammen Oxo bedeuten; $R^{10}$ und $R^{11}$ Wasserstoff oder $R^{10}$ und $R^{11}$ zusammen Oxo bedeuten; $R^{12}$ und $R^{13}$ Wasserstoff oder $R^{12}$ und $R^{13}$ zusammen Oxo bedeuten; und $R^{14}$ die vorstehend für $R^4$ angegebenen Bedeutungen aufweist und vorzugsweise für Wasserstoff, Niederalkyl, das durch Hydroxy substituiert sein kann, oder Niederalkoxycarbonyl steht; mit der Massgabe, dass zumindest eine Oxo-Gruppe in Form von $R^5$ und $R^6$, $R^8$ und $R^9$, $R^{10}$ und $R^{11}$ oder $R^{12}$ und $R^{13}$ anwesend ist; wird mit einem geeigneten Reduktionsmittel behandelt, das in der Lage ist, besagte Oxo-Gruppe(n) in (eine) Methylen-Gruppe(n) umzuwandeln; oder

b) eine Verbindung der Formel III

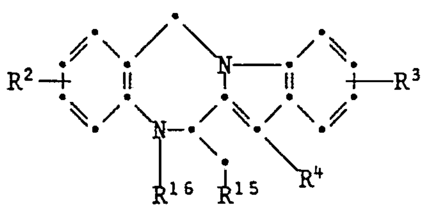

(III)

worin $R^{15}$ eine nukleophile Abgangsgruppe X bedeutet, $R^{16}$ die Gruppe $-CH_2-CH_2-NHR^7$ bedeutet, worin $R^7$ für Wasserstoff, Niederalkyl,$C_2$-$C_7$-Alkyl, welches durch Hydroxy, das durch eine Hydroxyschutzgruppe geschützt sein kann, substituiert ist, $C_2$-$C_7$-Alkyl, welches durch Amino oder Mononiederalkylamino, von denen jede Gruppe durch eine Aminoschutzgruppe geschützt ist, substituiert ist, $C_2$-$C_7$-Alkyl, welches durch Diniederalkylamino substituiert ist, steht, oder $R^7$ $C_3$-$C_7$-Alkenyl bedeutet, $C_3$-$C_7$-Alkinyl, oder 3 bis 7-gliedriges Cycloalkyl; oder eine Verbindung der Formel III, worin $R^{15}$ die Gruppe $-NHR^7$ bedeutet, worin $R^7$ wie vorstehend definiert ist und $R^{16}$ die Gruppe $-CH_2CH_2-X$ bedeutet, worin X für eine nukleophile Abgangsgruppe steht; oder eine Verbindung der Formel III, worin $R^{15}$ die Gruppe $-N(R^7)-CH_2CH_2-X$ bedeutet, worin die Substituenten die vorstehend erwähnten Bedeutungen aufweisen und $R^{16}$Wasserstoff bedeutet, oder ein reaktionsfähiges Derivat davon; und die anderen Substituenten die vorstehend angegebenen Bedeutungen aufweisen; wird intramolekular cyclisiert, und anwesende Schutzgruppen werden entfernt; oder

c) eine Verbindung der Formel IV

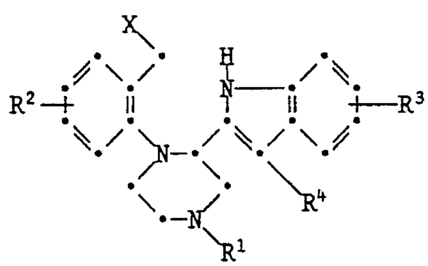

(IV)

worin X eine nukleophile Abgangsgruppe bedeutet und die anderen Substituenten die vorstehend angegebenen Bedeutungen aufweisen, oder ein reaktionsfähiges Derivat davon, wird intramolekular cyclisiert; und, wenn erwünscht, eine Verbindung, die nach einem der Verfahren a) bis c) erhalten wurde, wird in eine andere erfindungsgemässe Verbindung übergeführt, und/oder ein erhaltenes Salz wird in die freie Verbindung oder in ein anderes Salz übergeführt und/oder eine erhaltene freie Verbindung, die eine salzbildende Gruppe aufweist, wird in ein Salz übergeführt.

**19.** Verfahren gemäss Anspruch 18, gekennzeichnet durch die folgenden Verfahrensschritte: eine Verbindung, die nach einem der Verfahren a) bis c) erhalten wurde, wird in eine andere erfindungsgemässe Verbindung übergeführt, z.B. durch Veresterung, Amidierung, Decarboxylierung oder Reduktion von freiem Carboxy $R^4$, oder durch Ueberführung von verestertem oder amidiertem Carboxy $R^4$ in freies Carboxy, oder durch Umwandlung einer Verbindung der Formel I, worin $R^1$ Wasserstoff bedeutet, in eine Verbindung der Formel I, worin $R^1$ die vorstehenden Bedeutungen ausser Wasserstoff aufweist, oder durch Umwandeln einer Verbindung der Formel I, worin $R^1$ die vorstehend angegebenen Bedeutungen aufweist ausser Wasserstoff, in das 2-N-Oxid, oder durch Umwandeln des 2-N-Oxides einer Verbindung der Formel I in die entsprechende Verbindung der Formel I.

**Patentansprüche für folgende Vertragsstaaten : AT, ES, GR**

**1.** Verfahren zur Herstellung von Verbindungen der Formel I,

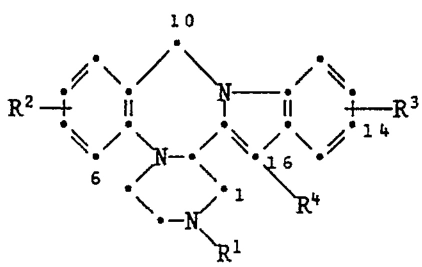

(I)

worin $R^1$ Wasserstoff, Niederalkyl, $C_3$-$C_7$-Alkenyl, welches über ein gesättigtes Kohlenstoffatom gebunden ist, $C_3$-$C_7$-Alkinyl, welches über ein gesättigtes Kohlenstoffatom gebunden ist, 3 bis 7-gliedriges Cycloalkyl, oder $C_2$-$C_7$-Alkyl, welches durch einen Substituenten ausgewählt aus der Gruppe bestehend aus Hydroxy, Amino, N-Mononiederalkylamino und N,N-Diniederalkylamino substituiert ist, wobei diese Substituenten vom Ringstickstoffatom durch mindestens 2 Kohlenstoffatome getrennt sind, bedeutet; oder worin $R^1$ Niederalkyl, welches durch einen Substituenten ausgewählt aus der Gruppe bestehend aus 3 bis 7-gliedrigem Cycloalkyl, Carboxy, Niederalkoxycarbonyl, Carbamoyl, N-Mononiederalkylcarbamoyl, N,N-Diniederalkylcarbamoyl und Niederalkanoyl substituiert ist, bedeutet; oder worin $R^1$ Niederalkyl, welches durch Phenyl oder Benzoyl substituiert ist, bedeutet, wobei jedes dieser Phenyl- oder Benzoylradikale unsubstituiert oder durch bis zu drei Substituenten ausgewählt aus der Gruppe bestehend aus Niederalkyl, Niederalkoxy, Niederalkylthio, Halogen und Trifluormethyl substituiert ist; worin $R^2$ und $R^3$, unabhängig voneinander, für Wasserstoff, Niederalkyl, Hydroxy, Niederalkoxy, Halogen oder Trifluormethyl stehen und $R^4$ für Wasserstoff, Niederalkyl, Hydroxyniederalkyl, Carboxy, Niederalkoxycarbonyl, Carbamoyl, N-Mono- oder N,N-Diniederalkylcarbamoyloder Formyl steht; wobei der Ausdruck "Nieder" Radikale mit bis zu und einschliesslich 7 Kohlenstoffatomen bezeichnet; Salzen, insbesondere pharmazeutisch annehmbaren Salzen davon; und den 2-N-Oxiden dieser Verbindungen, worin $R^1$ die vorstehend angegebenen Bedeutungen aufweist aber nicht Wasserstoff bedeutet, gekennzeichnet durch die folgenden Verfahrensschritte:

a) eine Verbindung der Formel II,

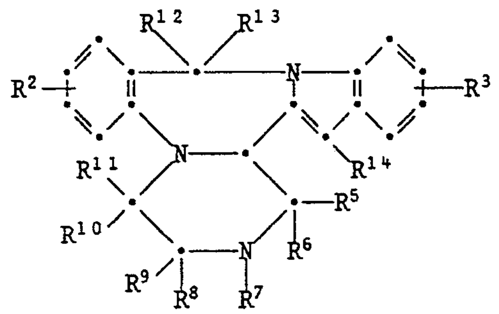

(II)

worin $R^2$ und $R^3$ die vorstehend angegebenen Bedeutungen aufweisen; $R^5$ und $R^6$ Wasserstoff bedeuten oder $R^5$ und $R^6$ zusammen Oxo bedeuten; $R^7$ die Bedeutung von $R^1$ hat und vorzugsweise für Wasserstoff, Niederalkyl, $C_2$-$C_7$-Alkyl, welches durch Hydroxy, Amino oder Mono- oder Diniederalkylamino substituiert ist, $C_3$-$C_7$-Alkenyl, $C_3$-$C_7$-Alkinyl, oder Niederalkyl, welches durch 3 bis 7-gliedriges Cycloalkyl oder durch gegebenenfalls substituiertes Phenyl substituiert ist, steht; $R^8$ und $R^9$ Wasserstoff bedeuten oder $R^8$ und $R^9$ zusammen Oxo bedeuten; $R^{10}$ und $R^{11}$ Wasserstoff oder $R^{10}$ und $R^{11}$ zusammen Oxo bedeuten; $R^{12}$ und $R^{13}$ Wasserstoff oder $R^{12}$ und $R^{13}$ zusammen Oxo bedeuten; und $R^{14}$ die vorstehend für $R^4$ angegebenen Bedeutungen aufweist und vorzugsweise für Wasserstoff, Niederalkyl, das durch Hydroxy substituiert sein kann, oder Niederalkoxycarbonyl steht; mit der Massgabe, dass zumindest eine Oxo-Gruppe in Form von $R^5$ und $R^6$, $R^8$ und $R^9$ , $R^{10}$ und $R^{11}$ oder $R^{12}$ und $R^{13}$ anwesend ist; wird mit einem geeigneten Reduktionsmittel behandelt, das in der Lage ist, besagte Oxo-Gruppe(n) in (eine) Methylen-Gruppe(n) umzuwandeln; oder

b) eine Verbindung der Formel III

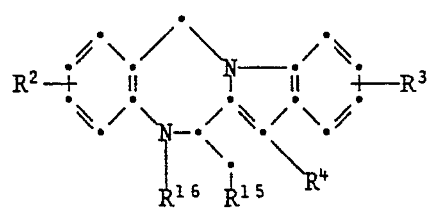

(III)

worin $R^{15}$ eine nukleophile Abgangsgruppe X bedeutet, $R^{16}$ die Gruppe -$CH_2$-$CH_2$-$NHR^7$ bedeutet, worin $R^7$ für Wasserstoff, Niederalkyl, $c_2$-$c_7$-Alkyl, welches durch Hydroxy, das durch eine Hydroxyschutzgruppe geschützt sein kann, substituiert ist, $C_2$-$C_7$-Alkyl, welches durch Amino oder Mononiederalkylamino, von denen jede Gruppe durch eine Aminoschutzgruppe geschützt ist, substituiert ist, $C_2$-$C_7$-Alkyl, welches durch Diniederalkylamino substituiert ist, steht, oder $R^7$ $C_3$-$C_7$-Alkenyl bedeutet, $C_3$-$C_7$-Alkinyl, oder 3 bis 7-gliedriges Cycloalkyl; oder eine Verbindung der Formel III, worin $R^{15}$ die Gruppe -$NHR^7$ bedeutet, worin $R^7$ wie vorstehend definiert ist und $R^{16}$ die Gruppe -$CH_2CH_2$-X bedeutet, worin X für eine nukleophile Abgangsgruppe steht; oder eine Verbindung der Formel III, worin $R^{15}$ die Gruppe -$N(R^7)$-$CH_2CH_2$-X bedeutet, worin die Substituenten die vorstehend erwähnten Bedeutungen aufweisen und $R^{16}$ Wasserstoff bedeutet, oder ein reaktionsfähiges Derivat davon; und die anderen Substituenten die vorstehend angegebenen Bedeutungen aufweisen; wird intramolekular cyclisiert, und anwesende Schutzgruppen werden entfernt; oder

c) eine Verbindung der Formel IV

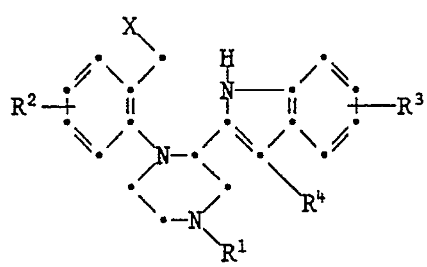

(IV)

worin X eine nukleophile Abgangsgruppe bedeutet und die anderen Substituenten die vorstehend angegebenen Bedeutungen aufweisen, oder ein reaktionsfähiges Derivat davon, wird intramolekular cyclisiert; und, wenn erwünscht, eine Verbindung, die nach einem der Verfahren a) bis c) erhalten wurde, wird in eine andere erfindungsgemässe Verbindung übergeführt, und/oder ein erhaltenes Salz wird in die freie Verbindung oder in ein anderes Salz übergeführt und/oder eine erhaltene freie Verbindung, die eine salzbildende Gruppe aufweist, wird in ein Salz übergeführt.

**2.** Verfahren gemäss Anspruch 1, gekennzeichnet durch die folgenden Verfahrensschritte: eine Verbindung, die nach einem der Verfahren a) bis c) erhalten wurde, wird in eine andere erfindungsgemässe Verbindung übergeführt, z.B. durch Veresterung, Amidierung, Decarboxylierung oder Reduktion von freiem Carboxy $R^4$, oder durch Ueberführung von verestertem oder amidiertem Carboxy $R^4$ in freies Carboxy, oder durch Umwandlung einer Verbindung der Formel I, worin $R^1$ Wasserstoff bedeutet, in eine Verbindung der Formel I, worin $R^1$ die vorstehenden Bedeutungen ausser Wasserstoff aufweist, oder durch Umwandeln einer Verbindung der Formel I, worin $R^1$ die vorstehend angegebenen Bedeutungen aufweist ausser Wasserstoff, in das 2-N-Oxid, oder durch Umwandeln des 2-N-Oxides einer Verbindung der Formel I in die entsprechende Verbindung der Formel I.

**3.** Verfahren zur Herstellung von Verbindungen der Formel I gemäss Anspruch 1, worin $R^1$ Wasserstoff, Niederalkyl, $C_3$-$C_7$-Alkenyl, welches über ein gesättigtes Kohlenstoffatom gebunden ist, oder $C_2$-$C_7$-Alkyl, welches durch Hydroxy substituiert ist, bedeutet; worin $R^2$ und $R^3$, unabhängig voneinander, für Wasserstoff, Niederalkyl, Niederalkoxy, Halogen oder Trifluormethyl stehen und $R^4$ für Wasserstoff, Niederalkyl, Hydroxymethyl, Carboxy, Niederalkoxycarbonyl, Carbamoyl, oder N-Mono-oder N,N-Diniederalkylcarbamoyl steht; pharmazeutisch annehmbaren Salzen davon; und den 2-N-Oxiden dieser Verbindungen, worin $R^1$ die vorstehend angegebenen Bedeutungen aufweist aber nicht Wasserstoff bedeutet.

**4.** Verfahren zur Herstellung von Verbindungen der Formel I gemäss Anspruch 1, worin $R^1$ Wasserstoff oder Niederalkyl bedeutet; $R^2$ für Wasserstoff, Niederalkyl, Halogen oder Trifluormethyl steht; $R^3$ für Wasserstoff, Niederalkyl, Niederalkoxy, Halogen oder Trifluormethyl steht; $R^4$ für Wasserstoff, Niederalkyl, Hydroxymethyl, Carboxy, Niederalkoxycarbonyl, Carbamoyl oder N-Mono-oder N,N-Diniederalkylcarbamoyl steht; und pharmazeutisch annehmbaren Salzen davon.

**5.** Verfahren zur Herstellung von Verbindungen der Formel I gemäss Anspruch 1, worin $R^1$ Wasserstoff oder Niederalkyl bedeutet; $R^2$ für Wasserstoff oder Halogen steht; $R^3$ für Wasserstoff, Niederalkoxy oder Halogen steht; $R^4$ für Carboxy, Niederalkoxycarbonyl, Carbamoyl oder N-Mono- oder N,N-Diniederalkylcarbamoyl steht; und pharmazeutisch annehmbaren Salzen davon.

**6.** Verfahren zur Herstellung von Verbindungen der Formel I gemäss Anspruch 1, worin $R^1$ Wasserstoff oder Niederalkyl bedeutet; $R^2$ für Wasserstoff oder Halogen steht; $R^3$ für Wasserstoff, Niederalkoxy oder Halogen steht; $R^4$ für Niederalkoxycarbonyl oder Carbamoyl steht; und pharmazeutisch annehmbaren Salzen davon.

**7.** Verfahren zur Herstellung von Verbindungen der Formel I gemäss Anspruch 1, worin $R^1$ Niederalkyl bedeutet; $R^2$ und $R^3$ für Wasserstoff stehen; $R^4$ für Niederalkoxycarbonyl steht; und pharmazeutisch annehmbaren Salzen davon.

**8.** Verfahren gemäss Anspruch 1 zur Herstellung von 1,3,4,16b-Tetrahydro-2-methyl-2H,10H-indolo[2,1-c]-pyrazino[1,2-a][1,4]benzodiazepin oder einem pharmazeutisch annehmbaren Salz davon.

**9.** Verfahren gemäss Anspruch 1 zur Herstellung von 1,3,4,16b-Tetrahydro-2-methyl-2H,10H-indolo[2,1-c]-pyrazino[1,2-a][1,4]benzodiazepin-16-carbonsäuremethylester oder einem pharmazeutisch annehmbaren Salz davon.

**10.** Verfahren zur Herstellung von Verbindungen der Formel IIA

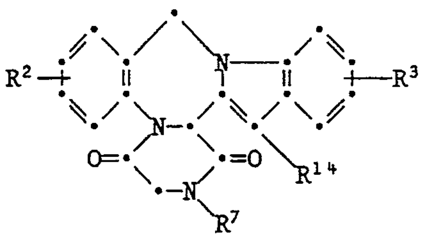

(IIA)

worin $R^7$ Wasserstoff, Niederalkyl, $C_3$-$C_7$-Alkenyl, welches über ein gesättigtes Kohlenstoffatom gebunden ist, $C_3$-$C_7$-Alkinyl, welches über ein gesättigtes Kohlenstoffatom gebunden ist, 3 bis 7-gliedriges Cycloalkyl, $C_2$-$C_7$-Alkyl, welches durch einen Substituenten ausgewählt aus der Gruppe bestehend aus Hydroxy, Amino, N-Mononiederalkylamino und N,N-Diniederalkylamino substituiert ist, wobei diese Substituenten vom Ringstickstoffatom durch mindestens 2 Kohlenstoffatome getrennt sind, bedeutet; oder worin $R^7$ Niederalkyl, welches durch einen Substituenten substituiert ist, welcher ausgewählt ist aus der Gruppe bestehend aus 3 bis 7-gliedrigem Cycloalkyl, Niederalkoxycarbonyl, Carbamoyl, und Phenyl, welches unsubstituiert oder durch bis zu 3 Substituenten ausgewählt aus der Gruppe Niederalkyl, Niederalkoxy, Niederalkylthio, Halogen und Trifluormethyl substituiert ist, bedeutet; $R^2$ und $R^3$ unabhängig voneinander Wasserstoff, Niederalkyl, Hydroxy, Niederalkoxy, Halogen oder Trifluormethyl bedeuten; und $R^{14}$ für Wasserstoff, Niederalkyl, Hydroxyniederalkyl, Niederalkoxycarbonyl oder Carbamoyl steht; wobei der Ausdruck "Nieder" Radikale mit bis zu und einschließlich 7 Kohlenstoffatomen bezeichnet; und Salzen davon, dadurch gekennzeichnet, dass ein Niederalkylester, wie ein Ethylester der Formel V,

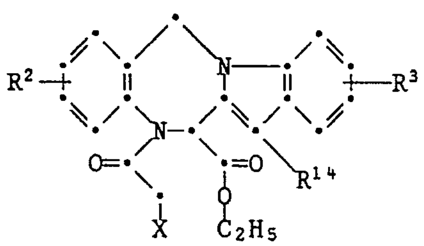

(V)

worin die Substituenten $R^2$, $R^3$ und $R^{14}$ die vorstehend angegebenen Bedeutungen aufweisen, X eine Abgangsgruppe bedeutet, wie Chlor, mit einem Amin der Formel $R^7$-$NH_2$, worin $R^7$ die vorstehend angegebenen Bedeutungen aufweist, mit der Massgabe, dass Amino, Niederalkylamino und, wenn notwendig, Hydroxygruppen, welche in $R^7$ anwesend sind, durch eine geeignete Amino- oder Hydroxyschutzgruppe geschützt sind und dass diese Schutzgruppen später entfernt werden, umgesetzt wird.

**11.** Verfahren zur Herstellung von Verbindungen der Formel IIA gemäss Anspruch 10, worin $R^7$ für Wasserstoff oder Niederalkyl steht, $R^2$ Wasserstoff, Niederalkyl, Halogen oder Trifluormethyl bedeutet, $R^3$ für Wasserstoff, Niederalkyl, Niederalkoxy, Halogen oder Trifluormethyl steht, und $R^{14}$ Wasserstoff, Niederalkyl oder Niederalkoxycarbonyl bedeutet, und Salze davon.

**12.** Verwendung der in den Ansprüchen 1 und 3 bis 9 genannten Verbindungen zur Herstellung von pharmazeutischen Präparaten.

**13.** Verwendung der in den Ansprüchen 1 und 3 bis 9 genannten Verbindungen zur Herstellung von pharmazeutischen Präparaten zur Verwendung als Serotonin-2-Rezeptor-Antagonisten.

**14.** Verfahren zur Herstellung eines pharmazeutischen Präparates, gekennzeichnet durch die Verarbeitung eines erfindungsgemässen Wirkstoffs nach einem der Ansprüche 1 und 3 bis 9 mit einem pharmazeutischen Trägermaterial.

**Claims**

**Claims for the following Contracting States : BE, CH, DE, FR, GB, IT, LI, LU, NL, SE.**

1. A compound of the formula I,

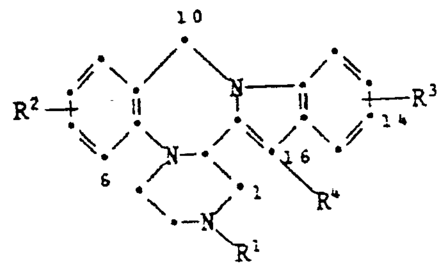

(I)

wherein $R^1$ is hydrogen, lower alkyl, $C_3$-$C_7$alkenyl bonded by way of a saturated carbon atom, $C_3$-$C_7$alkynyl bonded by way of a saturated carbon atom, 3- to 7-membered cycloalkyl, or $C_2$-$C_7$alkyl substituted by a substituent selected from the group consisting of hydroxy, amino, N-mono-lower alkylamino and N,N-di-lower alkylamino wherein said substituents are separated from the ring nitrogen atom by at least 2 carbon atoms; or $R^1$ is lower alkyl substituted by a substituent selected from the group consisting of 3- to 7-membered cycloalkyl, carboxy, lower alkoxycarbonyl, carbamoyl, N-mono-lower alkylcarbamoyl, N,N-di-lower alkylcarbamoyl and lower alkanoyl; or $R^1$ is lower alkyl substituted by phenyl or benzoyl, each of said phenyl or benzoyl radicals being unsubstituted or substituted by up to three substituents selected from the group consisting of lower alkyl, lower alkoxy, lower alkylthio, halogen and trifluoromethyl; $R^2$ and $R^3$, independently of one another, represent hydrogen, lower alkyl, hydroxy, lower alkoxy, halogen or trifluoromethyl and $R^4$ represents hydrogen, lower alkyl, hydroxy-lower alkyl, carboxy, lower alkoxycarbonyl, carbamoyl, N-mono-or N,N-di-lower alkylcarbamoyl, or formyl; the term "lower" designating radicals having up to and including 7 carbon atoms; a salt thereof, especially a pharmaceutically acceptable salt thereof; or the 2-N-oxide of any such compound wherein $R^1$ is as defined above but does not represent hydrogen.

2. A compound of formula I according to claim 1 wherein $R^1$ represents hydrogen, lower alkyl, $C_3$-$C_7$alkenyl bonded by way of a saturated carbon atom or $C_2$-$C_7$alkyl substituted by hydroxy; $R^2$ and $R^3$, independently of one another, represent hydrogen, lower alkyl, lower alkoxy, halogen or trifluoromethyl and $R^4$ represents hydrogen, lower alkyl, hydroxymethyl, carboxy, lower alkoxycarbonyl, carbamoyl or N-mono- or N,N-di-lower alkylcarbamoyl; or a pharmaceutically acceptable salt thereof; or the 2-N-oxide of any such compound wherein $R^1$ is as defined above but does not represent hydrogen.

3. A compound of formula I according to claim 1 wherein $R^1$ represents hydrogen or lower alkyl; $R^2$ represents hydrogen, lower alkyl, halogen or trifluoromethyl; $R^3$ represents hydrogen, lower alkyl, lower alkoxy, halogen or trifluoromethyl; $R^4$ represents hydrogen, lower alkyl, hydroxymethyl, carboxy, lower alkoxycarbonyl, carbamoyl or N-mono- or N,N-di-lower alkylcarbamoyl; or a pharmaceutically acceptable salt thereof.

4. A compound of formula I according to claim 1 wherein $R^1$ represents hydrogen or lower alkyl; $R^2$ represents hydrogen or halogen; $R^3$ represents hydrogen, lower alkoxy or halogen; $R^4$ represents carboxy, lower alkoxycarbonyl, carbamoyl or N-mono- or N-N-di-lower alkylcarbamoyl; or a pharmaceutically acceptable salt thereof.

5. A compound of formula I according to claim 1 wherein $R^1$ represents hydrogen or lower alkyl; $R^2$ represents hydrogen or halogen; $R^3$ represents hydrogen, lower alkoxy or halogen; $R^4$ represents lower alkoxycarbonyl or carbamoyl; or a pharmaceutically acceptable salt thereof.

6. A compound of formula I according to claim 1 wherein $R^1$ represents lower alkyl; $R^2$ and $R^3$ represent hydrogen; and $R^4$ represents lower alkoxycarbonyl; or a pharmaceutically acceptable salt thereof.

7. 1,3,4,16b-Tetrahydro-2-methyl-2H,10H-indolo-[2,1-c]pyrazino[1,2-a][1,4]benzodiazepine or a pharmaceutically acceptable salt thereof.

8. 1,3,4,16b-Tetrahydro-2-methyl-2H,10H-indolo-[2,1-c]pyrazino[1,2-a][1,4]benzodiazepine-16-carboxylic acid methyl ester or a pharmaceutically acceptable salt thereof.

**9.** A compound of the formula IIA

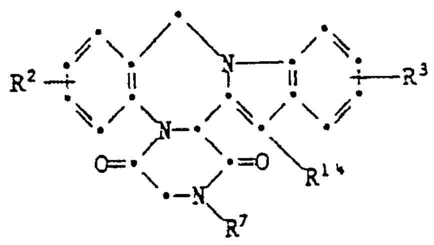

(IIA)

wherein $R^7$ is hydrogen, lower alkyl, $C_3$-$C_7$alkenyl bonded by way of a saturated carbon atom, $C_3$-$C_7$alkynyl bonded by way of a saturated carbon atom, 3- to 7-membered cycloalkyl, $C_2$-$C_7$alkyl substituted by a substituent selected from the group consisting of hydroxy, amino, N-mono-lower alkylamino and N,N-di-lower alkylamino wherein said substituents are separated from the ring nitrogen atom by at least 2 carbon atoms; or $R^7$ is lower alkyl substituted by a substituent selected from the group consisting of 3- to 7-membered cycloalkyl, lower alkoxycarbonyl, carbamoyl, and phenyl unsubstituted or substituted by up to three substituents selected from the group consisting of lower alkyl, lower alkoxy, lower alkylthio, halogen and trifluoromethyl; $R^2$ and $R^3$, independently of one another, represent hydrogen, lower alkyl, hydroxy, lower alkoxy, halogen or trifluoromethyl; and $R^{14}$ represents hydrogen, lower alkyl, hydroxy-lower alkyl, lower alkoxycarbonyl or carbamoyl, the term "lower" designating radicals having up to and including 7 carbon atoms, or a salt thereof.

**10.** A compound of formula IIA according to claim 9 wherein $R^7$ represents hydrogen or lower alkyl, $R^2$ represents hydrogen, lower alkyl, halogen or trifluoromethyl, $R^3$ represents hydrogen, lower alkyl, lower alkoxy, halogen or trifluoromethyl, and $R^{14}$ represents hydrogen, lower alkyl or lower alkoxycarbonyl, or a salt thereof.

**11.** A pharmaceutical composition comprising one or more of the compounds defined in claims 1 to 8.

**12.** A pharmaceutical composition comprising one or more of the compounds defined in claims 1 to 8 together with one or more pharmaceutically suitable carriers.

**13.** A compound defined in claims 1 to 8 for use in a method for the therapeutic treatment of the human or animal body.

**14.** A compound defined in claims 1 to 8 as a serotonin-2-receptor antagonist.

**15.** The use of a compound defined in claims 1 to 8 for the manufacture of a pharmaceutical composition.

**16.** The use of a compound defined in claims 1 to 8 for the manufacture of a pharmaceutical composition for use as a serotonin-2-receptor antagonist.

**17.** A process for the manufacture of a pharmaceutical composition, wherein a compound of the invention according to any one of claims 1 to 8 is processed with a pharmaceutical carrier.

**18.** A process for the manufacture of a compound of formula I according to claim 1, which comprises the following process steps:

a) a compound of the formula II

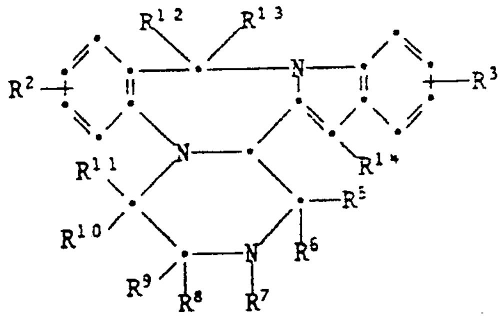

(II)

wherein $R^2$ and $R^3$ are as defined above; each of $R^5$ and $R^6$ represents hydrogen, or $R^5$ and $R^6$ together represent oxo; $R^7$ has the meaning of $R^1$ and is preferably hydrogen, lower alkyl, $C_2$-$C_7$alkyl substituted by hydroxy, amino or mono- or di-lower alkylamino, $C_3$-$C_7$alkenyl, $C_3$-$C_7$alkynyl, or lower alkyl which is substituted by 3- to 7-membered cycloalkyl or by optionally substituted phenyl; each of $R^8$ and $R^9$ represents hydrogen, or $R^8$ and $R^9$ together represent oxo; each of $R^{10}$ and $R^{11}$ represents hydrogen, or $R^{10}$ and $R^{11}$ together represent oxo; each of $R^{12}$ and $R^{13}$ represents hydrogen or $R^{12}$ and $R^{13}$ together represent oxo; and $R^{14}$ has the meanings of $R^4$ given above and is preferably hydrogen, lower alkyl which may be substituted by hydroxy, or lower alkoxycarbonyl; with the proviso that at least one oxo group represented by $R^5$ and $R^6$, $R^8$ and $R^9$, $R^{10}$ and $R^{11}$ or $R^{12}$, and $R^{13}$ is present; is treated with a suitable reducing agent capable of converting said oxo group(s) into (a) methylene group(s); or

b) a compound of the formula III

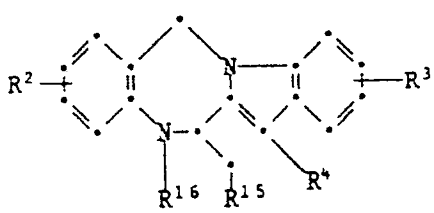

(III)

wherein $R^{15}$ represents a nucleophilic leaving group X, and $R^{16}$ represents the group $-CH_2-CH_2-NHR^7$ wherein $R^7$ represents hydrogen, lower alkyl, $C_2$-$C_7$alkyl substituted by hydroxy which may be protected by a hydroxy-protecting group, $C_2$-$C_7$alkyl substituted by amino or by mono-lower alkylamino each of which is protected by an amino-protecting group, or $C_2$-$C_7$alkyl substituted by di-lower alkylamino, or $R^7$ represents $C_3$-$C_7$alkenyl, $C_3$-$C_7$alkynyl or 3- to 7-membered cycloalkyl; or a compound of formula III wherein $R^{15}$ represents the group $-NHR^7$ wherein $R^7$ is as defined above and $R^{16}$ represents the group $-CH_2CH_2-X$ wherein X represents a nucleophilic leaving group; or a compound of formula III wherein $R^{15}$ represents the group $-N(R^7)-CH_2CH_2-X$ wherein the substituents have the meanings mentioned above and $R^{16}$ represents hydrogen, or a reactive derivative thereof; and the other substituents have the meanings given above; is intramolecularly cyclised, and protecting groups present are removed; or

c) a compound of the formula IV

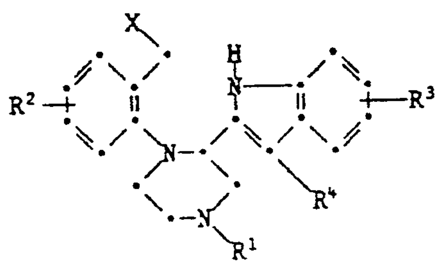

(IV)

wherein X represents a nucleophilic leaving group and the other substituents have the meanings

given above, or a reactive derivative thereof, is intramolecularly cyclised; and, if desired, a compound obtained by any one of the processes a) to c) is converted into another compound according to the invention, and/or a resulting salt is converted into the free compound or into another salt and/or a resulting free compound having a salt-forming group is converted into a salt.

**19.** A process according to claim 18 comprising the following process steps: a compound obtained by any one of processes a) to c) is converted into another compound according to the invention, for example by esterifying, amidating, decarboxylating or reducing free carboxy $R^4$, or by converting esterified or amidated carboxy $R^4$ into free carboxy, or by converting a compound of the formula I wherein $R^1$ is hydrogen into a compound of the formula I wherein $R^1$ has the meanings given above except hydrogen, or by converting a compound of the formula I wherein $R^1$ has the meanings given above, except hydrogen, into the 2-N-oxide, or by converting the 2-N-oxide of a compound of formula I into the corresponding compound of the formula I.

**Claims for the following Contracting States : AT, ES, GR.**

**1.** A process for the manufacture of a compound of the formula I,

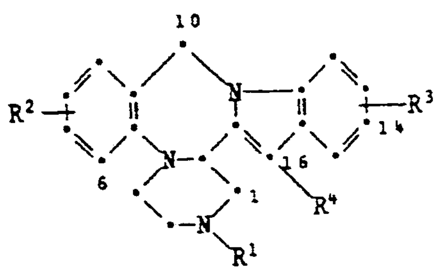

(I)

wherein $R^1$ is hydrogen, lower alkyl, $C_3$-$C_7$alkenyl bonded by way of a saturated carbon atom, $C_3$-$C_7$alkynyl bonded by way of a saturated carbon atom, 3- to 7-membered cycloalkyl, or $C_2$-$C_7$alkyl substituted by a substituent selected from the group consisting of hydroxy, amino, N-mono-lower alkylamino and N,N-di-lower alkylamino wherein said substituents are separated from the ring nitrogen atom by at least 2 carbon atoms; or $R^1$ is lower alkyl substituted by a substituent selected from the group consisting of 3- to 7-membered cycloalkyl, carboxy, lower alkoxycarbonyl, carbamoyl, N-mono-lower alkylcarbamoyl, N,N-di-lower alkylcarbamoyl and lower alkanoyl; or $R^1$ is lower alkyl substituted by phenyl or benzoyl, each of said phenyl or benzoyl radicals being unsubstituted or substituted by up to three substituents selected from the group consisting of lower alkyl, lower alkoxy, lower alkylthio, halogen and trifluoromethyl; $R^2$ and $R^3$, independently of one another, represent hydrogen, lower alkyl, hydroxy, lower alkoxy, halogen or trifluoromethyl and $R^4$ represents hydrogen, lower alkyl, hydroxy-lower alkyl, carboxy, lower alkoxycarbonyl, carbamoyl, N-mono-or N,N-di-lower alkylcarbamoyl, or formyl; the term "lower" designating radicals having up to and including 7 carbon atoms; a salt thereof, especially a pharmaceutically acceptable salt thereof; or the 2-N-oxide of any such compound wherein $R^1$ is as defined above but does not represent hydrogen, which process comprises the following process steps:

a) a compound of the formula II

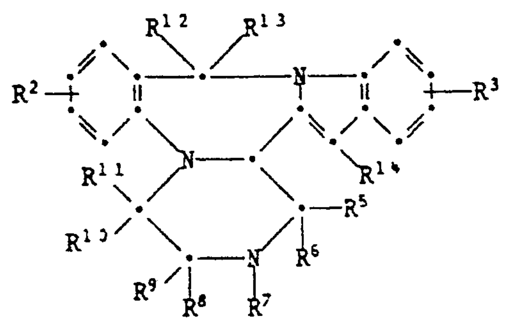

(II)

wherein $R^2$ and $R^3$ are as defined above; each of $R^5$ and $R^6$ represents hydrogen, or $R^5$ and $R^6$ together represent oxo; $R^7$ has the meaning of $R^1$ and is preferably hydrogen, lower alkyl, $C_2$-$C_7$alkyl substituted by hydroxy, amino or mono- or di-lower alkylamino, $C_3$-$C_7$alkenyl, $C_3$-$C_7$alkynyl, or lower alkyl which is substituted by 3- to 7-membered cycloalkyl or by optionally substituted phenyl; each of $R^8$ and $R^9$ represents hydrogen, or $R^8$ and $R^9$ together represent oxo; each of $R^{10}$ and $R^{11}$ represents hydrogen, or $R^{10}$ and $R^{11}$ together represent oxo; each of $R^{12}$ and $R^{13}$ represents hydrogen or $R^{12}$ and $R^{13}$ together represent oxo; and $R^{14}$ has the meanings of $R^4$ given above and is preferably hydrogen, lower alkyl which may be substituted by hydroxy, or lower alkoxycarbonyl; with the proviso that at least one oxo group represented by $R^5$ and $R^6$, $R^8$ and $R^9$, $R^{10}$ and $R^{11}$ or $R^{12}$ and $R^{13}$ is present; is treated with a suitable reducing agent capable of converting said oxo group(s) into (a) methylene group(s); or
b) a compound of the formula III

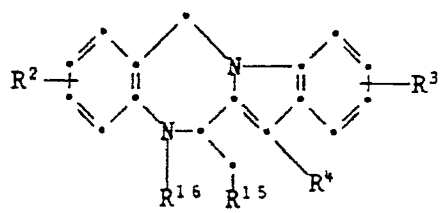

(III)

wherein $R^{15}$ represents a nucleophilic leaving group X, and $R^{16}$ represents the group $-CH_2-CH_2-NHR^7$ wherein $R^7$ represents hydrogen, lower alkyl, $C_2$-$C_7$alkyl substituted by hydroxy which may be protected by a hydroxy-protecting group, $C_2$-$C_7$alkyl substituted by amino or by mono-lower alkylamino each of which is protected by an amino-protecting group, or $C_2$-$C_7$alkyl substituted by di-lower alkylamino, or $R^7$ represents $C_3$-$C_7$alkenyl, $C_3$-$C_7$alkynyl or 3- to 7-membered cycloalkyl; or a compound of formula III wherein $R^{15}$ represents the group $-NHR^7$ wherein $R^7$ is as defined above and $R^{16}$ represents the group $-CH_2CH_2-X$ wherein X represents a nucleophilic leaving group; or a compound of formula III wherein $R^{15}$ represents the group $-N(R^7)-CH_2CH_2-X$ wherein the substituents have the meanings mentioned above and $R^{16}$ represents hydrogen, or a reactive derivative thereof; and the other substituents have the meanings given above; is intramolecularly cyclised, and protecting groups present are removed; or
c) a compound of the formula IV

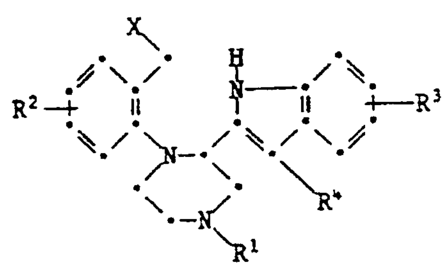

(IV)

wherein X represents a nucleophilic leaving group and the other substituents have the meanings given above, or a reactive derivative thereof, is intramolecularly cyclised; and, if desired, a compound obtained by any one of the processes a) to c) is converted into another compound according to the invention, and/or a resulting salt is converted into the free compound or into another salt and/or a resulting free compound having a salt-forming group is converted into a salt.

2. A process according to claim 1 comprising the following process steps: a compound obtained by any one of processes a) to c) is converted into another compound according to the invention, for example by esterifying, amidating, decarboxylating or reducing free carboxy $R^4$, or by converting esterified or amidated carboxy $R^4$ into free carboxy, or by converting a compound of the formula I wherein $R^1$ is hydrogen into a compound of the formula I wherein $R^1$ has the meanings given above except hydrogen, or by converting a compound of the formula I wherein $R^1$ has the meanings given above, except hydrogen, into the 2-N-oxide, or by converting the 2-N-oxide of a compound of formula I into the

corresponding compound of the formula I.

**3.** A process for the manufacture of a compound of formula I according to claim 1, wherein $R^1$ represents hydrogen, lower alkyl, $C_3$-$C_7$alkenyl bonded by way of a saturated carbon atom or $C_2$-$C_7$alkyl substituted by hydroxy; $R^2$ and $R^3$, independently of one another, represent hydrogen, lower alkyl, lower alkoxy, halogen or trifluoromethyl and $R^4$ represents hydrogen, lower alkyl, hydroxymethyl, carboxy, lower alkoxycarbonyl, carbamoyl or N-mono- or N,N-di-lower alkylcarbamoyl; or a pharmaceutically acceptable salt thereof; or the 2-N-oxide of any such compound wherein $R^1$ is as defined above but does not represent hydrogen.

**4.** A process for the manufacture of a compound of formula I according to claim 1, wherein $R^1$ represents hydrogen or lower alkyl; $R^2$ represents hydrogen, lower alkyl, halogen or trifluoromethyl; $R^3$ represents hydrogen, lower alkyl, lower alkoxy, halogen or trifluoromethyl; $R^4$ represents hydrogen, lower alkyl, hydroxymethyl, carboxy, lower alkoxycarbonyl, carbamoyl or N-mono- or N,N-di-lower alkylcarbamoyl; or a pharmaceutically acceptable salt thereof.

**5.** A process for the manufacture of a compound of formula I according to claim 1, wherein $R^1$ represents hydrogen or lower alkyl; $R^2$ represents hydrogen or halogen; $R^3$ represents hydrogen, lower alkoxy or halogen; $R^4$ represents carboxy, lower alkoxycarbonyl, carbamoyl or N-mono-or N,N-di-lower alkylcarbamoyl; or a pharmaceutically acceptable salt thereof.

**6.** A process for the manufacture of a compound of formula I according to claim 1, wherein $R^1$ represents hydrogen or lower alkyl; $R^2$ represents hydrogen or halogen; $R^3$ represents hydrogen, lower alkoxy or halogen; $R^4$ represents lower alkoxycarbonyl or carbamoyl; or a pharmaceutically acceptable salt thereof.

**7.** A process for the manufacture of a compound of formula I according to claim 1, wherein $R^1$ represents lower alkyl; $R^2$ and $R^3$ represent hydrogen; and $R^4$ represents lower alkoxycarbonyl; or a pharmaceutically acceptable salt thereof.

**8.** A process according to claim 1 for the manufacture of 1,3,4,16b-tetrahydro-2-methyl-2H,10H-indolo[2,1-c]-pyrazino[1,2-a][1,4]benzodiazepine or a pharmaceutically acceptable salt thereof.

**9.** A process according to claim 1 for the manufacture of 1,3,4,16b-tetrahydro-2-methyl-2H,10H-indolo[2,1-c]-pyrazino[1,2-a][1,4]benzodiazepine-16-carboxylic acid methyl ester or a pharmaceutically acceptable salt thereof.

**10.** A process for the manufacture of a compound of the formula IIA

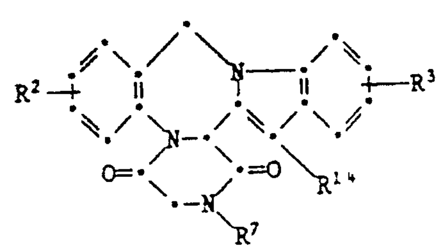

(IIA)

wherein $R^7$ is hydrogen, lower alkyl, $C_3$-$C_7$alkenyl bonded by way of a saturated carbon atom, $C_3$-$C_7$alkynyl bonded by way of a saturated carbon atom, 3- to 7-membered cycloalkyl, $C_2$-$C_7$alkyl substituted by a substituent selected from the group consisting of hydroxy, amino, N-mono-lower alkylamino and N,N-di-lower alkylamino wherein said substituents are separated from the ring nitrogen atom by at least 2 carbon atoms; or $R^7$ is lower alkyl substituted by a substituent selected from the group consisting of 3- to 7-membered cycloalkyl, lower alkoxycarbonyl, carbamoyl, and phenyl unsubstituted or substituted by up to three substituents selected from the group consisting of lower alkyl, lower alkoxy, lower alkylthio, halogen and trifluoromethyl; $R^2$ and $R^3$, independently of one another, represent hydrogen, lower alkyl, hydroxy, lower alkoxy, halogen or trifluoromethyl; and $R^{14}$

represents hydrogen, lower alkyl, hydroxy-lower alkyl, lower alkoxycarbonyl or carbamoyl, the term "lower" designating radicals having up to and including 7 carbon atoms, or a salt thereof, which process comprises reacting a lower alkyl ester, such as an ethyl ester of the formula V

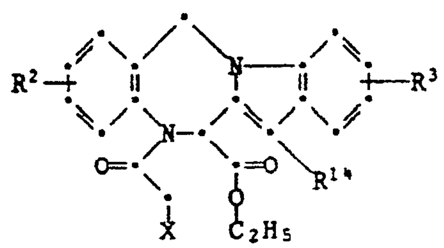

(V)

wherein the substituents $R^2$, $R^3$ and $R^{14}$ have the meanings given above and X represents a leaving group, such as chlorine, with an amine of the formula $R^7$-$NH_2$ wherein $R^7$ has the meanings given above, with the proviso that amino, lower alkylamino and, if necessary, hydroxy groups present in $R^7$ are protected by suitable amino- or hydroxy-protecting groups and said protecting groups are later removed.

**11.** A process for the manufacture of a compound of formula IIA according to claim 10 wherein $R^7$ represents hydrogen or lower alkyl, $R^2$ represents hydrogen, lower alkyl, halogen or trifluoromethyl, $R^3$ represents hydrogen, lower alkyl, lower alkoxy, halogen or trifluoromethyl, and $R^{14}$ represents hydrogen, lower alkyl or lower alkoxycarbonyl, or a salt thereof.

**12.** The use of a compound defined in claims 1 and 3 to 9 for the manufacture of a pharmaceutical composition.

**13.** The use of a compound defined in claims 1 and 3 to 9 for the manufacture of a pharmaceutical composition for use as a serotonin-2-receptor antagonist.

**14.** A process for the manufacture of a pharmaceutical composition, wherein a compound of the invention according to any one of claims 1 and 3 to 9 is processed with a pharmaceutical carrier.

## Revendications

**Revendications pour les Etats contractants suivants : BE, CH, DE, FR, GB, IT, LI, LU, NL, SE**

**1.** Composés de formule 1,

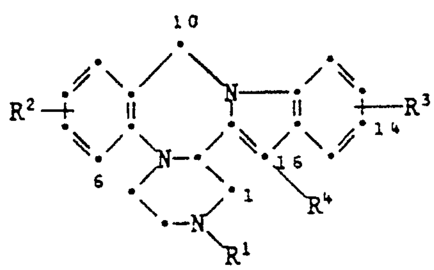

(I)

dans laquelle $R^1$ représente l'hydrogène, un groupe alkyle inférieur, alcényle en C 3-C 7 relié par l'intermédiaire d'un atome de carbone saturé, alcynyle en C 3-C 7 relié par l'intermédiaire d'un atome de carbone saturé, cycloalkyle de 3 à 7 chaînons ou alkyle en C 2-C 7 portant un substituant choisi parmi les groupes hydroxy, amino, N-mono-(alkyle inférieur)-amino et N,N-di-(alkyle inférieur)-amino, ces substituants étant séparés de l'atome d'azote cyclique par au moins deux atomes de carbone ; ou bien $R^1$ représente un groupe alkyle inférieur portant un substituant choisi parmi les groupes cycloalkyle de 3 à 7 chaînons, carboxy, (alcoxy inférieur)-carbonyle, carbamoyle, N-mono-(alkyle inférieur)-carbamoyle, N,N-di-(alkyle inférieur)-carbamoyle ou alcanoyle inférieur ; ou bien $R^1$ représente un groupe alkyle inférieur substitué par un groupe phényle ou benzoyle, chacun de ces radicaux

phényle ou benzoyle étant non substitué ou portant jusqu'à trois substituants choisis parmi les groupes alkyle inférieurs, alcoxy inférieurs, alkylthio inférieurs, les halogènes et les groupes trifluorométhyle ; $R^2$ et $R^3$ représentent chacun, indépendamment l'un de l'autre, l'hydrogène, un groupe alkyle inférieur, hydroxy, alcoxy inférieur, un halogène ou un groupe trifluorométhyle et $R^4$ représente l'hydrogène, un groupe alkyle inférieur, hydroxyalkyle inférieur, carboxy, (alcoxy inférieur)-carbonyle, carbamoyle, N-mono- ou N,N-di-(alkyle inférieur)-carbamoyle ou formyle, l'expression "inférieur" s'appliquant à des radicaux qui contiennent jusqu'à 7 atomes de carbone inclus ; leurs sels, en particulier leurs sels acceptables pour l'usage pharmaceutique ; et les 2-N-oxydes de ces composés pour lesquels $R^1$ a les significations indiquées ci-dessus à l'exception de l'hydrogène.

2. Composés de formule I selon la revendication 1, dans laquelle $R^1$ représente l'hydrogène, un groupe alkyle inférieur, alcényle inférieur en C 3-C 7 relié par l'intermédiaire d'un atome de carbone saturé ou un groupe alkyle en C 2-C 7 substitué par un groupe hydroxy ; $R^2$ et $R^3$ représentent chacun, indépendamment l'un de l'autre, l'hydrogène, un groupe alkyle inférieur, alcoxy inférieur, un halogène ou un groupe trifluorométhyle et $R^4$ représente l'hydrogène, un groupe alkyle inférieur, hydroxyméthyle, carboxy, (alcoxy inférieur)-carbonyle, carbamoyle ou N-mono- ou N,N-di-(alkyle inférieur)-carbamoyle ; leurs sels acceptables pour l'usage pharmaceutique et les 2-N-oxydes de ces composés pour lesquels $R^1$ a les significations indiquées ci-dessus à l'exception de l'hydrogène.

3. Composés de formule I selon la revendication 1, dans laquelle $R^1$ représente l'hydrogène ou un groupe alkyle inférieur ; $R^2$ représente l'hydrogène, un groupe alkyle inférieur, un halogène ou un groupe trifluorométhyle ; $R^3$ représente l'hydrogène, un groupe alkyle inférieur, alcoxy inférieur, un halogène ou un groupe trifluorométhyle ; $R^4$ représente l'hydrogène, un groupe alkyle inférieur, hydroxyméthyle. carboxy, (alcoxy inférieur)-carbonyle, carbamoyle ou N-mono- ou N,N-di-(alkyle inférieur)-carbamoyle ; et leurs sels acceptables pour l'usage pharmaceutique.

4. Composés de formule I selon la revendication 1, dans laquelle $R^1$ représente l'hydrogène ou un groupe alkyle inférieur ; $R^2$ représente l'hydrogène ou un halogène ; $R^3$ représente l'hydrogène, un groupe alcoxy inférieur ou un halogène ; $R^4$ représente un groupe carboxy, (alcoxy inférieur)-carbonyle, carbamoyle ou N-mono- ou N,N-di-(alkyle inférieur)-carbamoyle ; et leurs sels acceptables pour l'usage pharmaceutique.

5. Composés de formule I selon la revendication 1, dans laquelle $R^1$ représente l'hydrogène ou un groupe alkyle inférieur ; $R^2$ représente l'hydrogène ou un halogène ; $R^3$ représente l'hydrogène, un groupe alcoxy inférieur ou un halogène ; $R^4$ représente un groupe (alcoxy inférieur)-carbonyle ou carbamoyle ; et leurs sels acceptables pour l'usage pharmaceutique.

6. Composés de formule I selon la revendication 1, dans laquelle $R^1$ représente un groupe alkyle inférieur ; $R^2$ et $R^3$ représentent l'hydrogène ; $R^4$ représente un groupe (alcoxy inférieur)-carbonyle ; et leurs sels acceptables pour l'usage pharmaceutique.

7. La 1,3,4,16b-tétrahydro-2-méthyl-2H,10H-indolo[2,1-c]pyrazino[1,2-a][1,4]benzodiazépine ou un sel de ce composé acceptable pour l'usage pharmaceutique.

8. Le 1,3,4,16b-tétrahydro-2-méthyl-2H,10H-indolo[2,1-c]pyrazino[1,2-a][1,4]benzodiazépine-16-carboxylate de méthyle ou un sel acceptable pour l'usage pharmaceutique de ce composé.

9. Composés de formule IIA

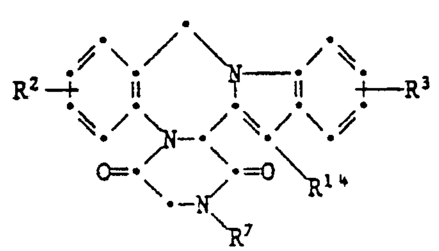

(IIA)

dans laquelle $R^7$ représente l'hydrogène, un groupe alkyle inférieur, alcényle en C 3-C 7 relié par l'intermédiaire d'un atome de carbone saturé, alcynyle en C 3-C 7 relié par l'intermédiaire d'un atome de carbone saturé, cycloalkyle de 3 à 7 chaînons, alkyle en C 2-C 7 portant un substituant choisi parmi les groupes hydroxy, amino, N-mono-(alkyle inférieur)-amino et N,N-di-(alkyle inférieur)-amino, ce substituant étant séparé de l'atome d'azote cyclique par au moins 2 atomes de carbone ;ou bien $R^7$ représente un groupe alkyle inférieur portant un substituant choisi parmi les groupes cycloalkyle de 3 à 7 chaînons, (alcoxy inférieur)-carbonyle, carbamoyle et phényle lui-même non substitué ou portant trois substituants choisis parmi les groupes alkyle inférieurs, alcoxy inférieurs, alkylthio inférieurs, les halogènes ou les groupes trifluorométhyle ; $R^2$ et $R^3$ représentent chacun, indépendamment l'un de l'autre, l'hydrogène, un groupe alkyle inférieur, hydroxy, alcoxy inférieur, un halogène ou un groupe trifluorométhyle ; et $R^{14}$ représente l'hydrogène, un groupe alkyle inférieur, hydroxyalkyle inférieur, (alcoxy inférieur)-carbonyle ou carbamoyle, l'expression "inférieur" s'appliquant à des radicaux qui contiennent jusqu'à 7 atomes de carbone inclus ; et leurs sels.

**10.** Composés de formule IIA de la revendication 9, dans laquelle $R^7$ représente l'hydrogène ou un groupe alkyle inférieur, $R^2$ représente l'hydrogène, un groupe alkyle inférieur, un halogène ou un groupe trifluorométhyle, $R^3$ représente l'hydrogène, un groupe alkyle inférieur, alcoxy inférieur, un halogène ou un groupe trifluorométhyle et $R^{14}$ représente l'hydrogène, un groupe alkyle inférieur ou (alcoxy inférieur)-carbonyle, et leurs sels.

**11.** Compositions pharmaceutiques contenant un ou plusieurs des composés mentionnés dans les revendications 1 à 8.

**12.** Compositions pharmaceutiques contenant un ou plusieurs des composés mentionnés dans les revendications 1 à 8 avec un ou plusieurs véhicules convenant pour l'usage pharmaceutique.

**13.** Les composés mentionnés dans les revendications 1 à 8, pour l'utilisation dans un procédé pour le traitement thérapeutique de l'organisme humain ou animal.

**14.** Les composés mentionnés dans les revendications 1 à 8, en tant qu'antagonistes des récepteurs de la sérotonine-2.

**15.** L'utilisation des composés mentionnés dans les revendications 1 à 8, pour la préparation de compositions pharmaceutiques.

**16.** L'utilisation des composés mentionnés dans les revendications 1 à 8, pour la préparation de compositions pharmaceutiques destinées à être utilisées en tant qu'antagonistes des récepteurs de la sérotonine-2.

**17.** Procédé de préparation d'une composition pharmaceutique, caractérisé en ce que l'on combine une substance active selon l'invention, selon l'une des revendications 1 à 8, avec un véhicule pharmaceutique.

**18.** Procédé de préparation des composés de formule I de la revendication 1, caractérisé par les stades opératoires suivants :

a) on traite un composé de formule II

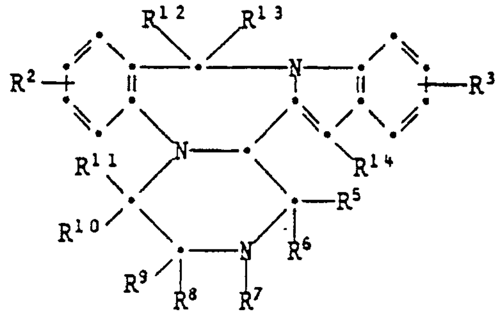

(II)

dans laquelle $R^2$ et $R^3$ ont les significations indiquées ci-dessus ; $R^5$ et $R^6$ représentent l'hydrogène ou bien, ensemble, un groupe oxo ; $R^7$ a les mêmes significations que $R^1$ et représente de préférence l'hydrogène, un groupe alkyle inférieur, un groupe alkyle en C 2-C 7 substitué par un groupe hydroxy, amino ou mono- ou di-(alkyle inférieur)-amino, un groupe alcényle en C 3-C 7, alcynyle en C 3-C 7 ou un groupe alkyle inférieur substitué par un groupe cycloalkyle de 3 à 7 chaînons ou par un groupe phényle lui-même éventuellement substitué ; $R^8$ et $R^9$ représentent l'hydrogène ou bien, ensemble, un groupe oxo ; $R^{10}$ et $R^{11}$ représentent l'hydrogène ou bien, ensemble, un groupe oxo ; $R^{12}$ et $R^{13}$ représentent l'hydrogène ou bien, ensemble, un groupe oxo ; et $R^{14}$ a les significations indiquées ci-dessus pour $R^4$ et représente de préférence l'hydrogène, un groupe alkyle inférieur qui peut être substitué par un groupe hydroxy, ou un groupe (alcoxy inférieur)-carbonyle ; sous réserve qu'il y ait au moins un groupe oxo représenté par $R^5$ et $R^6$, $R^8$ et $R^9$, $R^{10}$ et $R^{11}$ ou $R^{12}$ et $R^{13}$ : par un agent réducteur approprié capable de convertir ce ou ces groupes oxo en groupes méthylène ; ou bien

b) on soumet un composé de formule III

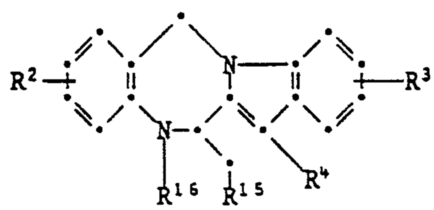

(III)

dans laquelle $R^{15}$ représente un substituant éliminable nucléophile X, $R^{16}$ représente le groupe $-CH_2-CH_2-NHR^7$ dans lequel $R^7$ représente l'hydrogène, un groupe alkyle inférieur, un groupe alkyle en C 2-C 7 substitué par un groupe hydroxy qui peut être protégé par un groupe protecteur approprié, un groupe alkyle en C 2-C 7 substitué par un groupe amino ou mono-(alkyle inférieur)-amino, chacun de ces groupes pouvant être protégé par un groupe protecteur approprié, un groupe alkyle en C 2-C 7 substitué par un groupe di-(alkyle inférieur)-amino, ou bien $R^7$ représente un groupe alcényle en C 3-C 7, alcynyle en C 3-C 7 ou cycloalkyle de 3 à 7 chaînons ; ou bien un composé de formule III dans laquelle $R^{15}$ représente le groupe $-NHR^7$ dans lequel $R^7$ a les significations indiquées ci-dessus et $R^{16}$ représente le groupe $-CH_2CH_2-X$ dans lequel X représente un substituant éliminable nucléophile ; ou bien un composé de formule III dans laquelle $R^{15}$ représente le groupe $-N(R^7)-CH_2CH_2-X$ dans lequel les symboles ont les significations indiquées ci-dessus et $R^{16}$ représente l'hydrogène, ou un dérivé réactif d'un tel composé ; et les autres symboles ont les significations indiquées ci-dessus ; à une cyclisation intramoléculaire, et on élimine les groupes protecteurs présents ; ou bien

c) on soumet un composé de formule IV

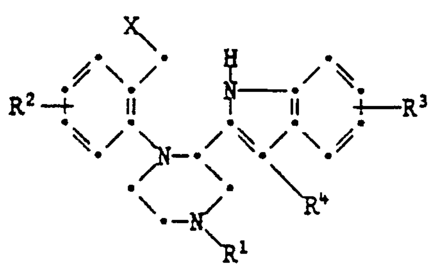

(IV)

dans laquelle X représente un substituant éliminable nucléophile et les autres symboles ont les significations indiquées ci-dessus, ou un dérivé réactif d'un tel composé, à une cyclisation intramoléculaire ; et si on le désire, on convertit un composé obtenu par l'un des procédés a) à c) en un autre composé selon l'invention et/ou on convertit un sel ainsi obtenu en le composé libre ou en un autre sel et/ou on convertit un composé obtenu à l'état libre et portant un groupe salifiable, en un sel.

**19.** Procédé selon la revendication 18, caractérisé par les stades opératoires suivants : on convertit un composé obtenu par l'un des procédés a) à c) en un autre composé selon l'invention, par exemple par

estérification, amidation, décarboxylation ou réduction du groupe carboxy libre $R^4$, ou par conversion du groupe carboxy estérifié ou amidé $R^4$ en un groupe carboxy libre, ou bien par conversion d'un composé de formule I dans laquelle $R^1$ représente l'hydrogène, en un composé de formule I dans laquelle $R^1$ a les significations indiquées ci-dessus à l'exception de l'hydrogène, ou par conversion d'un composé de formule I dans laquelle $R^1$ a les significations ci-dessus à l'exception de l'hydrogène, en le 2-N-oxyde, ou bien par conversion du 2-N-oxyde d'un composé de formule I en le composé correspondant de formule I.

**Revendications pour les Etats contractants suivants : AT, ES, GR**

**1.** Procédé de préparation des composés de formule I

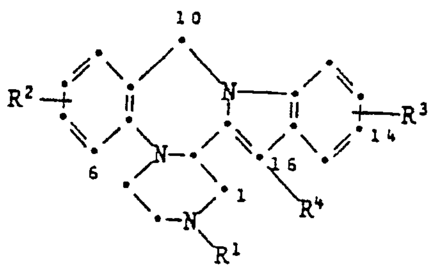

(I)

dans laquelle $R^1$ représente l'hydrogène, un groupe alkyle inférieur, alcényle en C 3-C 7 relié par l'intermédiaire d'un atome de carbone saturé, alcynyle en C 3-C 7 relié par l'intermédiaire d'un atome de carbone saturé, cycloalkyle de 3 à 7 chaînons ou alkyle en C 2-C 7 portant un substituant choisi parmi les groupes hydroxy, amino, N-mono-(alkyle inférieur)-amino et N,N-di-(alkyle inférieur)-amino, ces substituants étant séparés de l'atome d'azote cyclique par au moins deux atomes de carbone ; ou bien $R^1$ représente un groupe alkyle inférieur portant un substituant choisi parmi les groupes cycloalkyle de 3 à 7 chaînons, carboxy, (alcoxy inférieur)-carbonyle, carbamoyle, N-mono-(alkyle inférieur)-carbamoyle, N,N-di-(alkyle inférieur)-carbamoyle ou alcanoyle inférieur ; ou bien $R^1$ représente un groupe alkyle inférieur substitué par un groupe phényle ou benzoyle, chacun de ces radicaux phényle ou benzoyle étant non substitué ou portant jusqu'à trois substituants choisis parmi les groupes alkyle inférieurs, alcoxy inférieurs, alkylthio inférieurs, les halogènes et les groupes trifluorométhyle ; $R^2$ et $R^3$ représentent chacun, indépendamment l'un de l'autre, l'hydrogène, un groupe alkyle inférieur, hydroxy, alcoxy inférieur, un halogène ou un groupe trifluorométhyle et $R^4$ représente l'hydrogène, un groupe alkyle inférieur, hydroxyalkyle inférieur, carboxy, (alcoxy inférieur)-carbonyle, carbamoyle, N-mono- ou N,N-di-(alkyle inférieur)-carbamoyle ou formyle, l'expression "inférieur" s'appliquant à des radicaux qui contiennent jusqu'à 7 atomes de carbone inclus ; de leurs sels, en particulier leurs sels acceptables pour l'usage pharmaceutique : et des 2-N-oxydes de ces composés pour lesquels $R^1$ a les significations indiquées ci-dessus à l'exception de l'hydrogène, caractérisé par les stades opératoires suivants :

a) on traite un composé de formule II

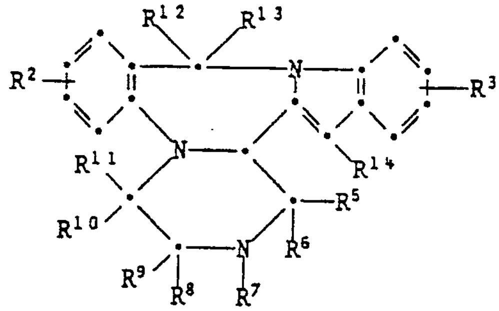

(II)

dans laquelle $R^2$ et $R^3$ ont les significations indiquées ci-dessus ; $R^5$ et $R^6$ représentent l'hydrogène ou bien, ensemble, un groupe oxo ; $R^7$ a les mêmes significations que $R^1$ et représente de

préférence l'hydrogène, un groupe alkyle inférieur, un groupe alkyle en C 2-C 7 substitué par un groupe hydroxy, amino ou mono- ou di-(alkyle inférieur)-amino, un groupe alcényle en C 3-C 7, alcynyle en C 3-C 7 ou un groupe alkyle inférieur substitué par un groupe cycloalkyle de 3 à 7 chaînons ou par un groupe phényle lui-même éventuellement substitué ; $R^8$ et $R^9$ représentent l'hydrogène ou bien, ensemble, un groupe oxo ; $R^{10}$ et $R^{11}$ représentent l'hydrogène ou bien, ensemble, un groupe oxo ; $R^{12}$ et $R^{13}$ représentent l'hydrogène ou bien, ensemble, un groupe oxo ; et $R^{14}$ a les significations indiquées ci-dessus pour $R^4$ et représente de préférence l'hydrogène, un groupe alkyle inférieur qui peut être substitué par un groupe hydroxy, ou un groupe (alcoxy inférieur)-carbonyle ; sous réserve qu'il y ait au moins un groupe oxo représenté par $R^5$ et $R^6$, $R^8$ et $R^9$, $R^{10}$ et $R^{11}$ ou $R^{12}$ et $R^{13}$ ; par un agent réducteur approprié capable de convertir ce ou ces groupes oxo en groupes méthylène ; ou bien

b) on soumet un composé de formule III

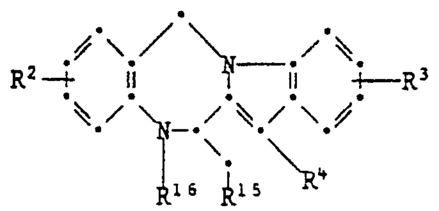

(III)

dans laquelle $R^{15}$ représente un substituant éliminable nucléophile X, $R^{16}$ représente le groupe $-CH_2-CH_2-NHR^7$ dans lequel $R^7$ représente l'hydrogène, un groupe alkyle inférieur, un groupe alkyle en C 2-C 7 substitué par un groupe hydroxy qui peut être protégé par un groupe protecteur approprié, un groupe alkyle en C 2-C 7 substitué par un groupe amino ou mono-(alkyle inférieur)-amino, chacun de ces groupes pouvant être protégé par un groupe protecteur approprié, un groupe alkyle en C 2-C 7 substitué par un groupe di-(alkyle inférieur)-amino, ou bien $R^7$ représente un groupe alcényle en C 3-C 7, alcynyle en C 3-C 7 ou cycloalkyle de 3 à 7 chaînons ; ou bien un composé de formule III dans laquelle $R^{15}$ représente le groupe $-NHR^7$ dans lequel $R^7$ a les significations indiquées ci-dessus et $R^{16}$ représente le groupe $-CH_2CH_2-X$ dans lequel X représente un substituant éliminable nucléophile ; ou bien un composé de formule III dans laquelle $R^{15}$ représente le groupe $-N(R^7)-CH_2CH_2-X$ dans lequel les symboles ont les significations indiquées ci-dessus et $R^{16}$ représente l'hydrogène, ou un dérivé réactif d'un tel composé ; et les autres symboles ont les significations indiquées ci-dessus ; à une cyclisation intromoléculaire, et on élimine les groupes protecteurs présents ; ou bien

c) on soumet un composé de formule IV

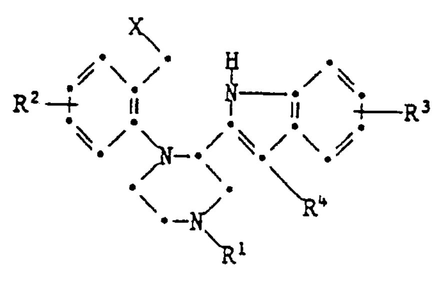

(IV)

dans laquelle X représente un substituant éliminable nucléophile et les autres symboles ont les significations indiquées ci-dessus, ou un dérivé réactif d'un tel composé, à une cyclisation intramoléculaire ; et si on le désire, on convertit un composé obtenu par l'un des procédés a) à c) en un autre composé selon l'invention et/ou on convertit un sel ainsi obtenu en le composé libre ou en un autre sel et/ou on convertit un composé obtenu à l'état libre et portant un groupe salifiable, en un sel.

2. Procédé selon la revendication 1, caractérisé par les stades opératoires suivants : on convertit un composé obtenu par l'un des procédés a) à c) en un autre composé selon l'invention, par exemple par estérifiaction, amidation, décarboxylation ou réduction du groupe carboxy libre $R^4$, ou par conversion du groupe carboxy estérifié ou amidé $R^4$ en un groupe carboxy libre, ou bien par conversion d'un

composé de formule I dans laquelle $R^1$ représente l'hydrogène, en un composé de formule I dans laquelle $R^1$ a les significations indiquées ci-dessus à l'exception de l'hydrogène, ou par conversion d'un composé de formule I dans laquelle $R^1$ a les significations ci-dessus à l'exception de l'hydrogène, en le 2-N-oxyde, ou bien par conversion du 2-N-oxyde d'un composé de formule I en le composé correspondant de formule I.

**3.** Procédé de préparation des composés de formule I de la revendication 1, dans laquelle $R^1$ représente l'hydrogène, un groupe alkyle inférieur, alcényle inférieur en C 3-C 7 relié par l'intermédiaire d'un atome de carbone saturé ou un groupe alkyle en C 2-C 7 substitué par un groupe hydroxy ; $R^2$ et $R^3$ représentent chacun, indépendamment l'un de l'autre, l'hydrogène, un groupe alkyle inférieur, alcoxy inférieur, un halogène ou un groupe trifluorométhyle et $R^4$ représente l'hydrogène, un groupe alkyle inférieur, hydroxyméthyle, carboxy, (alcoxy inférieur)-carbonyle, carbamoyle ou N-mono- ou N,N-di-(alkyle inférieur)-carbamoyle ; leurs sels acceptables pour l'usage pharmaceutique et les 2-N-oxydes de ces composés pour lesquels $R^1$ a les significations indiquées ci-dessus à l'exception de l'hydrogène.

**4.** Procédé de préparation des composés de formule I de la revendication 1, dans laquelle $R^1$ représente l'hydrogène ou un groupe alkyle inférieur ; $R^2$ représente l'hydrogène, un groupe alkyle inférieur, un halogène ou un groupe trifluorométhyle ; $R^3$ représente l'hydrogène, un groupe alkyle inférieur, alcoxy inférieur, un halogène ou un groupe trifluorométhyle ; $R^4$ représente l'hydrogène, un groupe alkyle inférieur, hydroxyméthyle, carboxy, (alcoxy inférieur)-carbonyle, carbamoyle ou N-mono- ou N,N-di-(alkyle inférieur)-carbamoyle ; et leurs sels acceptables pour l'usage pharmaceutique.

**5.** Procédé de préparation des composés de formule I de la revendication 1, dans laquelle $R^1$ représente l'hydrogène ou un groupe alkyle inférieur ; $R^2$ représente l'hydrogène ou un halogène ; $R^3$ représente l'hydrogène, un groupe alcoxy inférieur ou un halogène ; $R^4$ représente un groupe carboxy, (alcoxy inférieur)-carbonyle, carbamoyle ou N-mono- ou N,N-di-(alkyle inférieur)-carbamoyle ; et leurs sels acceptables pour l'usage pharmaceutique.

**6.** Procédé de préparation des composés de formule I de la revendication 1, dans laquelle $R^1$ représente l'hydrogène ou un groupe alkyle inférieur ; $R^2$ représente l'hydrogène ou un halogène ; $R^3$ représente l'hydrogène, un groupe alcoxy inférieur ou un halogene ; $R^4$ représente un groupe (alcoxy inférieur)-carbonyle ou carbamoyle ; et leurs sels acceptables pour l'usage pharmaceutique.

**7.** Procédé de préparation des composés de formule I de la revendication 1, dans laquelle $R^1$ représente un groupe alkyle inférieur ; $R^2$ et $R^3$ représentent l'hydrogène ; $R^4$ représente un groupe (alcoxy inférieur)-carbonyle ; et leurs sels acceptables pour l'usage pharmaceutique.

**8.** Procédé selon la revendication 1, pour préparer la 1,3,4,16b-tétrahydro-2-méthyl-2H,10H-indolo-[2,1-c]-pyrazino[1,2-a][1,4]benzodiazépine ou un sel de ce composé acceptable pour l'usage pharmaceutique.

**9.** Procédé selon la revendication 1, pour préparer le 1,3,4,16b-tétrahydro-2-méthyl-2H,10H-indolo-[2,1-c]-pyrazino[1,2-a][1,4]benzodiazépine-16-carboxylate de méthyle ou un sel de ce composé acceptable pour l'usage pharmaceutique.

**10.** Procédé de préparation des composés de formule IIA

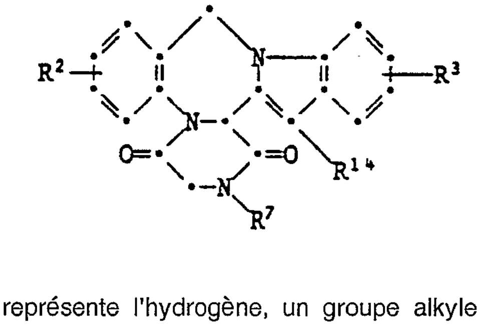

(IIA)

dans laquelle $R^7$ représente l'hydrogène, un groupe alkyle inférieur, alcényle en C 3-C 7 relié par

l'intermédiaire d'un atome de carbone saturé, alcynyle en C 3-C 7 relié par l'intermédiaire d'un atome de carbone saturé, cycloalkyle de 3 à 7 chaînons, alkyle en C 2-C 7 portant un substituant choisi parmi les groupes hydroxy, amino, N-mono-(alkyle inférieur)-amino et N,N-di-(alkyle inférieur)-amino, ce substituant étant séparé de l'atome d'azote cyclique par au moins 2 atomes de carbone ; ou bien $R^7$ représente un groupe alkyle inférieur portant un substituant choisi parmi les groupes cycloalkyle de 3 à 7 chaînons, (alcoxy inférieur)-carbonyle, carbamoyle et phényle lui-même non substitué ou portant trois substituants choisis parmi les groupes alkyle inférieurs, alcoxy inférieurs, alkylthio inférieurs, les halogènes ou les groupes trifluorométhyle ; $R^2$ et $R^3$ représentent chacun, indépendamment l'un de l'autre, l'hydrogène, un groupe alkyle inférieur, hydroxy, alcoxy inférieur, un halogène ou un groupe trifluorométhyle ; et $R^{14}$ représente l'hydrogène, un groupe alkyle inférieur, hydroxyalkyle inférieur, (alcoxy inférieur)-carbonyle ou carbamoyle, l'expression "inférieur" s'appliquant à des radicaux qui contiennent jusqu'à 7 atomes de carbone inclus ; et leurs sels, caractérisé en ce que l'on fait réagir un ester alkylique inférieur, par exemple un ester éthylique de formule V

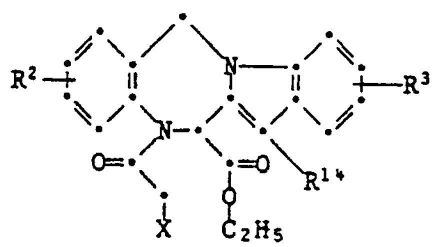

(V)

dans laquelle les symboles $R^2$ , $R^3$ et $R^{14}$ ont les significations indiquées ci-dessus et X représente un substituant éliminable tel que le chlore, avec une amine de formule $R^7$-$NH_2$ dans laquelle $R^7$ a les significations indiquées ci-dessus sous réserve que les groupes amino, alkylamino inférieurs et, lorsque c'est nécessaire, les groupes hydroxy, présents dans $R^7$, sont protégés par des groupes protecteurs appropriés, et on élimine ensuite ces groupes protecteurs.

**11.** Procédé de préparation des composés de formule IIA de la revendication 10, dans laquelle $R^7$représente l'hydrogène ou un groupe alkyle inférieur, $R^2$ représente l'hydrogène, un groupe alkyle inférieur, un halogène ou un groupe trifluorométhyle, $R^3$ représente l'hydrogène, un groupe alkyle inférieur, alcoxy inférieur, un halogène ou un groupe trifluorométhyle et $R^{14}$ représente l'hydrogène, un groupe alkyle inférieur ou (alcoxy inférieur)-carbonyle, et de leurs sels.

**12.** Utilisation des composés mentionnés dans les revendications 1 et 3 à 9, pour la préparation de compositions pharmaceutiques.

**13.** Utilisation des composés mentionnés dans les revendications 1 et 3 à 9, pour la préparation de compositions pharmaceutiques servant d'antagonistes pour les récepteurs de la sérotonine-2.

**14.** Procédé de préparation d'une composition pharmaceutique, caractérisé en ce que l'on combine une substance active selon l'invention selon l'une des revendications 1 et 3 à 9, avec un véhicule pharmaceutique.